(19) Europäisches Patentamt
European Patent Office
Office européen des brevets

(11) **EP 3 626 169 B1**

(12) **EUROPEAN PATENT SPECIFICATION**

(45) Date of publication and mention of the grant of the patent:
**29.03.2023 Bulletin 2023/13**

(21) Application number: **17910174.6**

(22) Date of filing: **15.05.2017**

(51) International Patent Classification (IPC):
**A61B 5/11** (2006.01)     **A61B 5/103** (2006.01)
**A61B 5/00** (2006.01)

(52) Cooperative Patent Classification (CPC):
**A61B 5/1036; A61B 5/112; A61B 5/4064; A61B 5/4082; A61B 5/6823; A61B 5/6828; A61B 5/6829;** A61B 2503/08; A61B 2503/10; A61B 2562/0219

(86) International application number:
**PCT/JP2017/018148**

(87) International publication number:
**WO 2018/211550 (22.11.2018 Gazette 2018/47)**

(54) **INFORMATION PROCESSING DEVICE, INFORMATION PROCESSING SYSTEM, AND INFORMATION PROCESSING METHOD**

INFORMATIONSVERARBEITUNGSVORRICHTUNG, INFORMATIONSVERARBEITUNGSSYSTEM UND INFORMATIONSVERARBEITUNGSVERFAHREN

DISPOSITIF DE TRAITEMENT D'INFORMATIONS, SYSTÈME DE TRAITEMENT D'INFORMATIONS ET PROCÉDÉ DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.03.2020 Bulletin 2020/13**

(73) Proprietor: **Fujitsu Limited**
**Kawasaki-shi, Kanagawa 211-8588 (JP)**

(72) Inventors:
• **WASHIZAWA, Shiho**
**Kawasaki-shi**
**Kanagawa 211-8588 (JP)**
• **HOTTA, Shinji**
**Kawasaki-shi**
**Kanagawa 211-8588 (JP)**

• **SASAMOTO, Yuki**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake Kempner LLP**
**Cheapside House**
**138 Cheapside**
**London EC2V 6BJ (GB)**

(56) References cited:
EP-A2- 1 466 557     JP-A- 2008 173 365
JP-A- 2009 261 595     JP-A- 2011 078 534
JP-A- 2011 177 278     JP-A- 2012 037 245
JP-A- 2016 106 948     US-A1- 2013 110 010

**Description**

TECHNICAL FIELD

The present invention relates to an information processing apparatus, an information processing system, and an information processing method.

BACKGROUND ART

In recent years, a system has been developed that assists a medical worker by quantifying daily conditions of a patient by using a wearable sensor and the like. Specifically, for example, a technology is known that quantitatively evaluates the state of the patient by continuously recognizing a walking condition of the patient.

In addition, conventionally, a technology is known that calculates a feature amount regarding walking disorder by using a locus of a waist calculated from an acceleration sensor attached to the waist with respect to a walking section extracted by a motion sensor and determines a walking disorder degree by comparing the feature amount with data that has been learned in advance.

Patent document US 2013/110010 A1 relates to a gait analysis device including a measuring unit configured to measure a subject's motion; a determining unit configured to determine a walking start point in time at which the subject starts walking based on the subject's motion; a feature quantity calculator configured to, when the walking start point in time is determined, calculate a feature quantity of the subject's motion measured during a predetermined time period starting from the walking start point in time as a time period in which the subject's motion is not stabilized; and an estimating unit configured to estimate a subject's walking condition based on the feature quantity.

Patent document EP 1466557 A2 relates to a physical movement analyzer for performing analysis on postures and movements based on physical feature amounts that are extracted at the time when a human moves, especially relates to a physical movement analyzer or the like that is used for a walking ability test in a rehabilitation system or a sport training system.

Patent document JP 2016 106848 A relates to a step count measuring apparatus for measuring the number of steps of a user.

CITATION LIST

PATENT DOCUMENT

Patent Document 1: Japanese Laid-open Patent Publication No. 2010-110399
Patent Document 2: Japanese Laid-open Patent Publication No. 2011-177278

SUMMARY OF INVENTION

TECHNICAL PROBLEM

In the conventional technology described above, the walking section in which the walking disorder is determined is not limited. Therefore, conventionally, when the walking section is extracted, in a case where a section in which disorder does not appear is extracted as a walking section, there is a possibility to wrongly determine that the disorder does not occur even though the patient has walking disorder in actual.

An object of the disclosed technology is to improve accuracy of estimation of a physical state.

SOLUTION TO PROBLEM

The present invention is defined by the independent claims, to which reference should now be made. Specific embodiments are defined in the dependent claims.

Each of the units in embodiments can be a procedure that implements each unit, a program that causes a computer to execute each unit as processing, and a computer readable recording medium storing the program.

ADVANTAGEOUS EFFECTS OF INVENTION

Accuracy of estimation of a physical state can be improved.

BRIEF DESCRIPTION OF DRAWINGS

FIG. 1 is a diagram for explaining an outline of an information processing system according to a first embodiment.

FIG. 2 is a diagram illustrating an exemplary system configuration of the information processing system according to the first embodiment.

FIG. 3 is a diagram for explaining a hardware configuration of an information processing apparatus according to the first embodiment.

FIG. 4 is a diagram illustrating an example of a load critical point database according to the first embodiment.

FIG. 5 is a diagram illustrating an example of a model database according to the first embodiment.

FIG. 6 is a diagram for explaining a function of an abnormal walking estimation processing unit according to the first embodiment.

FIG. 7 is a flowchart for explaining processing of the abnormal walking estimation processing unit according to the first embodiment.

FIG. 8 is a diagram for explaining specification of a walking section by a walking section specification unit according to the first embodiment.

FIG. 9 is a flowchart for explaining processing of a load section extraction unit according to the first embodiment.

FIG. 10 is a diagram for explaining exemplary determination data according to the first embodiment.

FIG. 11 is a diagram for explaining specification of a section by a section specification unit according to the first embodiment.

FIG. 12 is a diagram for explaining processing by a feature amount calculation unit according to the first embodiment.

FIG. 13 is a flowchart for explaining processing for calculating a moving width of a waist by an upper body feature amount calculation unit according to the first embodiment.

FIG. 14 is a flowchart for explaining processing for calculating an angle of the waist by the upper body feature amount calculation unit according to the first embodiment.

FIG. 15 is a diagram for explaining motions of legs of a subject according to the first embodiment.

FIG. 16 is a flowchart for explaining processing for calculating a leg feature amount by a leg feature amount calculation unit according to the first embodiment.

FIG. 17 is a diagram for explaining calculation of a regression coefficient by a relationship calculation unit according to the first embodiment.

FIG. 18 is a first diagram for explaining determination of a wobble by a wobble determination unit according to the first embodiment.

FIG. 19 is a second diagram for explaining the determination of the wobble by the wobble determination unit according to the first embodiment.

FIG. 20 is a diagram for explaining a relation between a wobble of an upper body and movements of both legs.

FIG. 21 is a diagram for explaining estimation by an abnormal walking estimation unit according to the first embodiment.

FIG. 22 is a diagram illustrating an exemplary output of estimation result information according to the first embodiment.

FIG. 23 is a flowchart for explaining processing of a critical point database creation unit according to the first embodiment.

FIG. 24 is a first diagram for explaining creation of the load critical point database according to the first embodiment.

FIG. 25 is a second diagram for explaining the creation of the load critical point database according to the first embodiment.

FIG. 26 is a third diagram for explaining the creation of the load critical point database according to the first embodiment.

FIG. 27 is a flowchart for explaining processing of a model database creation unit according to the first embodiment.

FIG. 28 is a diagram for explaining creation of the model database according to the first embodiment.

FIG. 29 is a diagram for explaining a function of an abnormal walking estimation processing unit according to a second embodiment.

FIG. 30 is a diagram for explaining a relation between normal walking and repetition of wobbles according to the second embodiment.

FIG. 31 is a flowchart for explaining processing of the abnormal walking estimation processing unit according to the second embodiment.

FIG. 32 is a diagram illustrating an exemplary output of estimation result information according to the second embodiment.

FIG. 33 is a diagram illustrating an exemplary system configuration of an information processing system according to a third embodiment.

DESCRIPTION OF EMBODIMENTS

(First Embodiment)

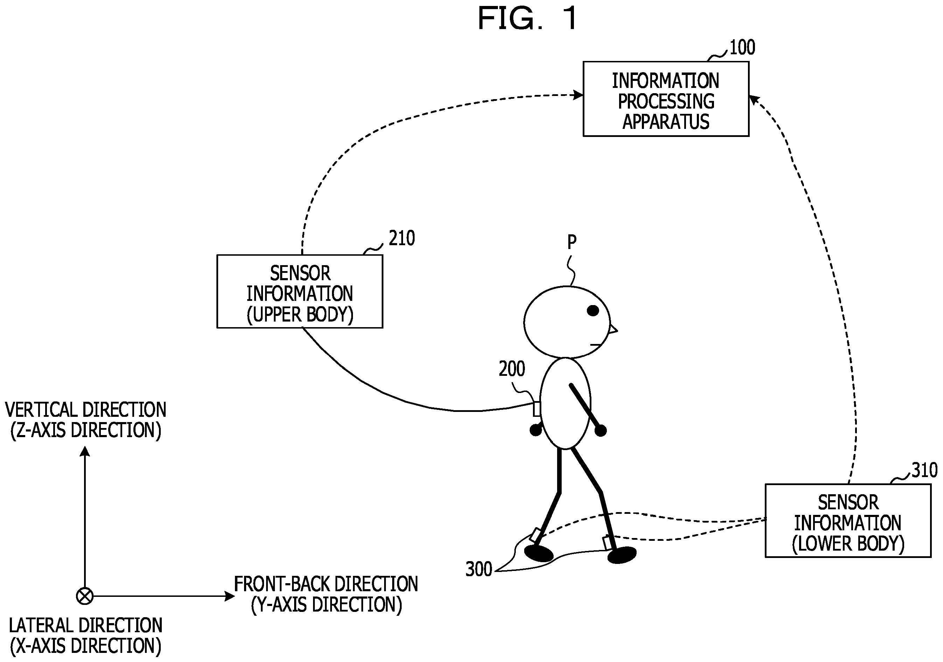

Hereinafter, a first embodiment will be described with reference to the drawings. FIG. 1 is a diagram for explaining an outline of an information processing system according to the first embodiment.

In the information processing system according to the present embodiment, an information processing apparatus 100 acquires sensor information 210 and 310 respectively from a sensor 200 attached to an upper body of a subject P whose walking state (physical state) is estimated and a sensor 300 attached to a lower body of the subject P. Then, the information processing apparatus 100 estimates the walking state of the subject P based on the sensor information 210 and 310. More specifically, the information processing apparatus 100 according to the present embodiment estimates whether or not an abnormal motion occurs while the subject P is walking. Therefore, the information processing apparatus 100 according to the present embodiment plays a role of an abnormal walking estimation apparatus that estimates whether or not an abnormality occurs while the subject P is walking.

Here, the walking state and the abnormality in the walking state in the present embodiment will be described.

The walking state in the present embodiment indicates a state where the subject P is walking. Furthermore, the abnormality in the walking state in the present embodiment indicates a wobble during walking, a movement of the upper body that is not seen in a normal walking motion, and the like.

One of factors that causes abnormalities during walking is a concussion. As one of main symptoms of the concussion, a decrease in balance ability is known. Furthermore, it is known that a concussion patient tends to wobble to the left and right when walking a distance that a healthy person can walk straight due to the decrease in the balance ability.

Moreover, the inventor of the present application has found that a concussion patient tends to be unbalanced in a case where a physical load such as long-time walking is applied.

Therefore, in the present embodiment, this point is focused. By extracting a section in which the subject P is walking in a state where the physical load is applied from the walking section of the subject P and estimating whether or not an abnormality occurs during walking based on a feature of a motion of the subject P in this section, accuracy of the estimation is improved.

Note that in the present embodiment, whether or not the abnormality occurs during walking is estimated mainly based on the decrease in the balance ability of the concussion patient. However, it is not necessary for the subject P who is a target of estimation whether or not the abnormality occurs to be a concussion patient.

The information processing apparatus 100 according to the present embodiment acquires information indicating the feature of the motion of the subject P based on the sensor information 210 and 310 detected by the sensors 200 and 300 and estimates whether or not the abnormality occurs during walking based on the information indicating the feature of the motion.

Furthermore, in the present embodiment, the upper body to which the sensor 200 is attached indicates a portion of the body of the subject P upper than the waist, and the lower body indicates a portion of the body of the subject P lower than the waist. Note that the waist is a region connecting the upper body and the lower body.

The sensor 200 according to the present embodiment is, for example, an inertial sensor such as an acceleration sensor. It is sufficient that the sensor 200 be attached to the upper body of the subject P. However, the sensor 200 is attached near the waist so as to acquire the sensor information 210 indicating a motion of the entire upper body. Note that the sensor 200 may also be attached to a chest, or the like. Furthermore, the sensor 200 according to the present embodiment may include a sensor that acquires a pulse rate, positional information, and the like of the subject P who wears the sensor 200. For example, the sensor 200 may be mounted on a terminal device such as a smartphone held by the subject P.

It is sufficient that the sensor information 210 according to the present embodiment include an acceleration signal, and the sensor information 210 may include, for example, a signal acquired by the sensor 200 such as a signal indicating a pulse other, in addition to the acceleration signal.

The sensor 300 according to the present embodiment may be, for example, an inertial sensor such as a gyro sensor. In the present embodiment, in a case where the sensor 300 is the gyro sensor, it is sufficient that the sensor be attached to the lower body of the subject P. However, it is preferable that the sensor 300 be attached to each of left and right ankles so as to acquire the sensor information 310 indicating the motions of both legs of the lower body. It is sufficient that the sensor information 310 according to the present embodiment include an angular speed signal.

Furthermore, in the following description, a sagittal plane that is provided in parallel to the median line of the body of the subject P and divides the body into left and right portions is referred to as a Y-Z plane, and the Z-axis direction on the sagittal plane is referred to as a vertical direction, and the Y-axis direction is referred to as a front-back direction. Note that it is preferable that the sagittal plane be a median sagittal plane that equally divides the body into the left and the right portions along the median line. However, planes separated rightward and leftward and parallel to the median line are also the sagittal plane.

Furthermore, in the following description, a cross section that is a plane perpendicular to the body axis of the subject P is referred to as an X-Y plane, and the X-axis direction on the cross section is referred to as a lateral direction. Furthermore, a coronal plane indicating an arbitrary plane that divides the body of the subject P into a front side (ventral side) and a rear side (dorsal side) is an X-Z plane.

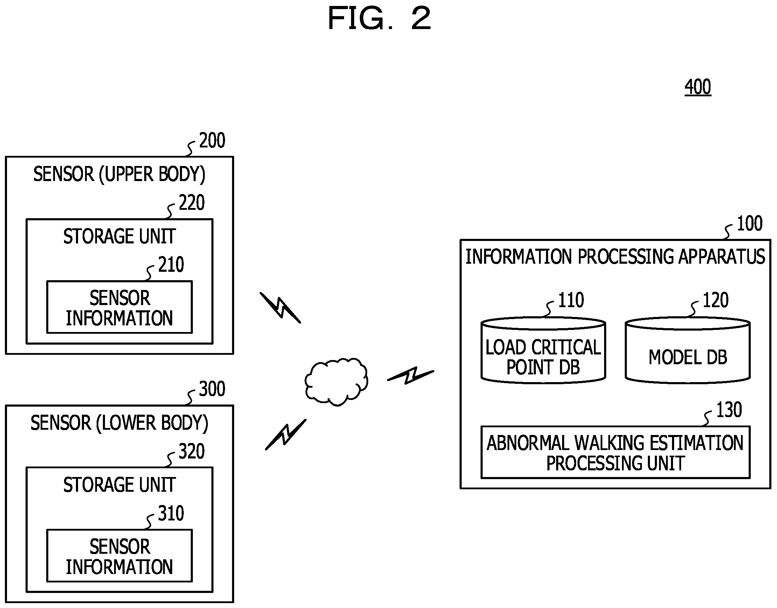

Next, an information processing system according to the present embodiment will be described with reference to FIG. 2. FIG. 2 is a diagram illustrating an exemplary system configuration of the information processing system according to the first embodiment.

An information processing system 400 according to the present embodiment includes the information processing apparatus 100, the sensor 200, and the sensor 300.

In the information processing system 400 according to the present embodiment, the information processing apparatus 100, the sensor 200, and the sensor 300 may be connected via a network and the like. Furthermore, the information processing apparatus 100 may be connected to the sensors 200 and 300 by wire or the like based on a predetermined communication standard.

The information processing apparatus 100 according to the present embodiment includes a load critical point database 110, a model database 120, and an abnormal walking estimation processing unit 130.

The load critical point database 110 stores information that is referred when a section in which a physical load to the subject P reaches a critical point is specified.

Note that the critical point in the present embodiment is a value indicating a limit of a range in which a concussion patient can maintain a physical normal state and in which a concussion patient strongly tends to be unbalanced. Therefore, in other words, the load critical point database 110 stores a threshold that is referred when it is determined whether or not a load of a predetermined size that strengthens the tendency to unbalance the subject P is applied to the subject P.

The model database 120 according to the present embodiment includes model parameters obtained from the feature of the motion in walking in a case where the load exceeds the critical point for each of a concussion patient group and a healthy person group. The model parameter is a parameter, for example, a mean vector, a covariance matrix, and the like. Details of each database will be described later.

The abnormal walking estimation processing unit 130 according to the present embodiment estimates the walking state of the subject P who wears the sensor 200 that detects the sensor information 210 based on the sensor information 210 and each database.

The sensor 200 according to the present embodiment includes a storage unit 220 that stores the sensor information 210 detected by the sensor 200. Furthermore, the sensor 300 of the present embodiment includes a storage unit 320 that stores the sensor information 310 detected by the sensor 300.

In the present embodiment, for example, the subject P makes some actions for a predetermined period in a state of attaching the sensors 200 and 300, and the sensor information 210 and 310 detected by the sensors 200 and 300 during the period may be held in the storage units 220 and 320 respectively. Additionally, after the sensors 200 and 300 are detached from the subject P, the pieces of sensor information 210 and 310 stored in the storage units 220 and 320 may be transmitted to the information processing apparatus 100.

The information processing apparatus 100 according to the present embodiment may be, for example, a computer provided in a medical institution, or the like. In the present embodiment, for example, when a patient who has a medical examination at a medical institution and a patient who has been hospitalized returns home, it is possible to attach the sensors 200 and 300 to the patient and assumes the patient as the subject P. Then, for example, when the subject P visits the medical institution and the like, the information processing apparatus 100 may acquire the sensor information 210 and 310 respectively stored in the storage units 220 and 320 from the sensors 200 and 300.

In the present embodiment, by using of the information processing system 400 as described above, for example, motions of a patient like an elderly person having a motion function needed to be carefully observed can be grasped by a medical worker or the like not only based on a subjective view of the patient but also based on an objective view. Moreover, in the present embodiment, it can be estimated whether or not an abnormality in the walking state of the subject P occurs. Therefore, according to the present embodiment, for example, when a medical worker determines whether or not a concussion patient is likely to recover, a degree of recovery, and the like, the determination can be assisted.

Note that in FIG. 2, a configuration is used in which the load critical point database 110 and the model database 120 are provided in the information processing apparatus 100. However, the configuration is not limited to this. The load critical point database 110 and the model database 120 may be provided in a storage device external to the information processing apparatus 100 and the like.

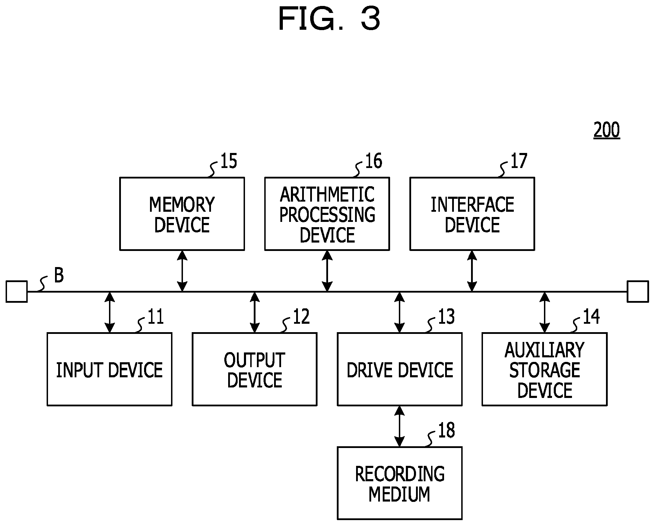

Next, a hardware configuration of the information processing apparatus 100 according to the present embodiment will be described with reference to FIG. 3. FIG. 3 is a diagram for explaining the hardware configuration of the information processing apparatus according to the first embodiment.

The information processing apparatus 100 according to the present embodiment includes an input device 11, an output device 12, a drive device 13, an auxiliary storage device 14, a memory device 15, an arithmetic processing device 16, and an interface device 17 that are connected to each other via a bus B.

The input device 11 is used to input various kinds of information and is implemented by, for example, a keyboard, a mouse, and the like. The output device 12 is used to output various information and is implemented by, for example, a display and the like. The interface device 17 includes a modem, a LAN card, and the like, and is used for connection to a network.

An information processing program is at least a part of various programs for controlling the information processing apparatus 100 and includes an abnormal walking estimation program that implements the abnormal walking estimation processing unit 130. The information processing program is provided by, for example, distribution of a recording medium 18, download from the network, and the like. As the recording medium 18 that records the information processing program, it is possible to use various types of recording media such as recording media that optically, electrically, or magnetically records information such as a CD-ROM, a flexible disk, or a magneto-optical disc, a semiconductor memory that electrically records information such as a ROM or a flash memory, and the like.

Additionally, the information processing program is installed in the auxiliary storage device 14 from the recording medium 18 via the drive device 13 when the recording medium 18 that records the information processing program is set in the drive device 13. The information processing program downloaded from the network is installed in the auxiliary storage device 14 via the interface device 17.

The auxiliary storage device 14 stores the installed information processing program and also stores necessary files, data, and the like. The memory device 15 reads and stores the information processing program from the auxiliary storage device 14 at the time of staring the computer. Then, the arithmetic processing device 16 executes various kinds of processing as described later in accordance with the information processing program stored in the memory device 15.

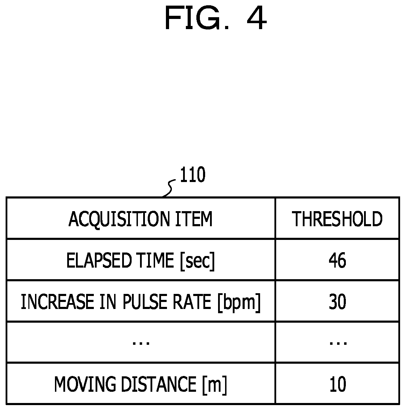

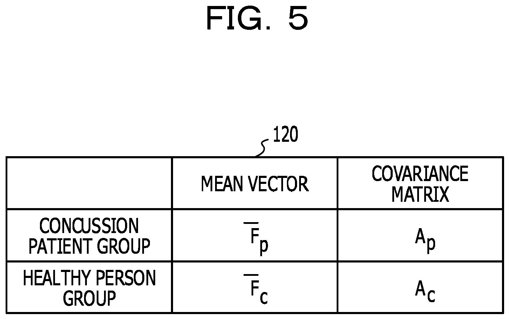

Next, the load critical point database 110 and the model database 120 included in the information processing apparatus 100 according to the present embodiment will be described with reference to FIGs. 4 and 5. FIG. 4 is a diagram illustrating an example of a load critical point database according to the first embodiment.

The load critical point database 110 according to the present embodiment is generated in advance and stored in the information processing apparatus 100. A method for generating the load critical point database 110 will be described later in detail.

The load critical point database 110 includes an acquisition item and a threshold as information items. A value of the item "acquisition item" indicates an information item acquired from the sensor information 210. Furthermore, a value of the item "acquisition item" according to the present embodiment indicates an item of which the value changes with time.

In the example in FIG. 4, as the acquisition item, an elapsed time, an increase in the pulse rate, a moving distance, and the like are included. In a case where the acquisition item is the "elapsed time", it is found that determination is made such that a physical load reaches the critical point when the elapsed time from the start of walking is 46 seconds. In other words, it can be said that a concussion patient is more likely to be unbalanced when 46 seconds have passed after the start of walking. Note that the elapsed time can be calculated from the sensor information 210. Furthermore, the elapsed time may be calculated from the sensor information 310 and information from a third sensor attached to an arm and the like.

Furthermore, in the example in FIG. 4, in a case where the acquisition item is the "increase in the pulse rate", it is found that the determination is made such the physical load reaches the critical point in a case where the pulse rate is increased from a value at the start of walking by equal to or more than 30 [bpm]. In other words, it can be said that a concussion patient is more likely to be unbalanced in a case where the pulse rate is increased from the value at the start by equal to or more than 30 [bpm].

Note that the value of the item "acquisition item" according to the present embodiment may include various items in addition to the elapsed time, the increase in the pulse rate, and the moving distance.

Specifically, for example, the number of times of wobbles from the start of walking, occurrence of an event (falling after start of walking and impact is added) and content of the event, whether or not environment is changed (change of floor state such as change from concrete to gravel road, change in surrounding illuminance), content of the environment, and the like may be included.

Next, the model database 120 will be described with reference to FIG. 5. FIG. 5 is a diagram illustrating an example of a model database according to the first embodiment.

The model database 120 according to the present embodiment is generated in advance and is provided in the information processing apparatus 100. A method for generating the model database 120 will be described later in detail.

In the model database 120 according to the present embodiment, a mean vector is associated with a covariance matrix for each of the concussion patient group and the healthy person group.

In the present embodiment, the mean vector indicates an average of an upper body feature amount indicating the feature of the motion of the upper body and a leg feature amount indicating the feature of the motions of both legs in a case where a person is walking in a state where the load exceeds the critical point. Note that the upper body feature amount in the present embodiment includes, for example, a plurality of values indicating the motion of the upper body such as an average and a standard deviation of the moving width of the waist in the vertical direction during walking, an average and a standard deviation of the moving width of the waist in the lateral direction during walking, and the like. Furthermore, the leg feature amount in the present embodiment includes a plurality of values indicating the motions of both legs such as an average value, a standard deviation, and the like of a walking speed, an average value, a standard deviation, and the like of a length of stride. The upper body feature amount and the leg feature amount will be described later in detail.

The mean vector of the concussion patient group is a set of the average values of the upper body feature amounts and the leg feature amounts of the plurality of concussion patients, and the mean vector of the healthy person group is a set of the average values of the upper body feature amounts and the leg feature amounts of the plurality of healthy people. Furthermore, the covariance matrix in the present embodiment is calculated from the mean vector of the concussion patient group and the mean vector of the healthy person group.

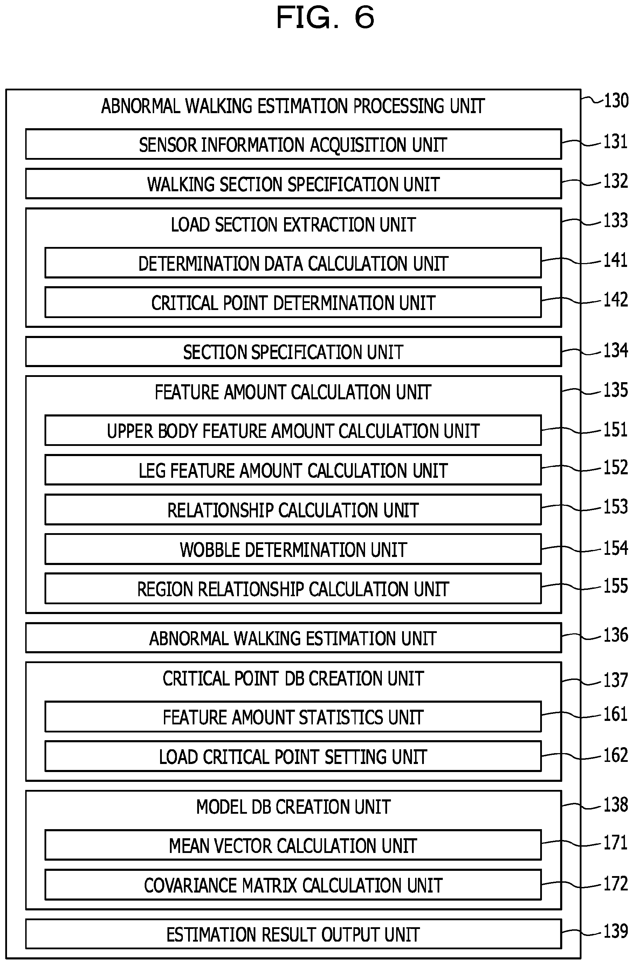

Next, a function of the abnormal walking estimation processing unit 130 included in the information processing apparatus 100 according to the present embodiment will be described with reference to FIG. 6. FIG. 6 is a diagram for explaining a function of an abnormal walking estimation processing unit according to the first embodiment.

The abnormal walking estimation processing unit 130 according to the present embodiment includes a sensor information acquisition unit 131, a walking section specification unit 132, a load section extraction unit (action section extraction unit) 133, a section specification unit 134, a feature amount calculation unit 135, an abnormal walking estimation unit 136, a critical point database creation unit 137, a model database creation unit 138, and an estimation result output unit 139.

The abnormal walking estimation processing unit 130 according to the present embodiment is implemented by executing the information processing program stored in the memory device 15 and the like by the arithmetic processing device 16 of the information processing apparatus 100.

The sensor information acquisition unit 131 according to the present embodiment acquires the sensor information 210 from the sensor 200.

The walking section specification unit 132 extracts a section in which the subject P is walking from the sensor information 210. Processing of the walking section specification unit 132 will be described later in detail.

The load section extraction unit 133 refers to the load critical point database 110 and extracts a walking section in which a physical load on the subject P reaches the critical point from a walking section specified by the walking section specification unit 132. The load section extraction unit 133 will be described later in detail.

The section specification unit 134 specifies a section in which the feature amount calculation unit 135 calculates a feature amount in the walking section extracted by the load section extraction unit 133. Specifically, for example, the section specification unit 134 may specify a section from a time when the load reaches the critical point to an end time of the walking section and the like as the section in which the feature amount is calculated, in the walking section extracted by the load section extraction unit 133.

The feature amount calculation unit 135 calculates the upper body feature amount indicating the feature of the motion of the upper body of the subject P and the leg feature amount indicating the motion of the lower body (both legs). The feature amount calculation unit 135 will be described later in detail.

The abnormal walking estimation unit 136 estimates whether or not an abnormality occurs while the subject P is walking based on the upper body feature amount and the leg feature amount calculated by the feature amount calculation unit 135 and the model database 120. Processing of the abnormal walking estimation unit 136 will be described later in detail.

The critical point database creation unit 137 sets a threshold for each item "acquisition item" based on the upper body feature amount and the leg feature amount calculated by the feature amount calculation unit 135 and generates the load critical point database 110. The critical point database creation unit 137 will be described later in detail.

The model database creation unit 138 generates the model database 120 based on the upper body feature amount and the leg feature amount in the section specified by the section specification unit 134. The model database creation unit 138 will be described later in detail.

The estimation result output unit 139 outputs an estimation result by the abnormal walking estimation unit 136. The estimation result output unit 139 according to the present embodiment may display information indicating the estimation result on a screen of the information processing apparatus 100 as the estimation result and may transmit the information indicating the estimation result to the outside of the information processing apparatus 100. In the following description, the information indicating the estimation result is referred to as estimation result information.

Note that the estimation result output unit 139 according to the present embodiment may include information indicating a degree of recovery of the subject P as the estimation result information of the subject P.

Next, the load section extraction unit 133 according to the present embodiment will be described. The load section extraction unit 133 according to the present embodiment includes a determination data calculation unit 141 and a critical point determination unit 142.

The determination data calculation unit 141 according to the present embodiment calculates a value indicated by the item "acquisition item" in the load critical point database 110 from the sensor information 210. In other words, the determination data calculation unit 141 calculates data used to determine whether or not the physical load on the subject P reaches the critical point. Specifically, for example, the determination data calculation unit 141 calculates the elapsed time after the subject P starts to walk, the increase in the pulse rate for each step, and the like from the sensor information 210.

The critical point determination unit 142 according to the present embodiment compares the threshold for each item "acquisition item" in the load critical point database 110 and the value of the item "acquisition item" calculated by the determination data calculation unit 141 and determines whether or not the load on the subject P reaches the critical point.

The load section extraction unit 133 according to the present embodiment extracts a walking section in which it is determined that the load has reached the critical point by the critical point determination unit 142 as a load section (action section) in which the physical load on the subject P exceeds the critical point.

Next, the feature amount calculation unit 135 according to the present embodiment will be described. The feature amount calculation unit 135 according to the present embodiment includes an upper body feature amount calculation unit 151, a leg feature amount calculation unit 152, a relationship calculation unit 153, a wobble determination unit 154, and a region relationship calculation unit 155.

The upper body feature amount calculation unit 151 according to the present embodiment calculates the upper body feature amount indicating the feature of the motion of the upper body of the subject P from the sensor information 210.

Specifically, the upper body feature amount calculation unit 151 acquires various feature amounts indicating the features of the motion of the upper body of the subject P based on the sensor information 210 and takes statistics of the feature amounts. Then, the upper body feature amount calculation unit 151 assumes the result of the statistics of various feature amounts as the upper body feature amount.

The leg feature amount calculation unit 152 according to the present embodiment calculates the leg feature amount indicating the feature of the motion of the lower body (both legs) of the subject P from the sensor information 310.

Specifically, the leg feature amount calculation unit 152 acquires various feature amounts indicating the features of the motion of the lower body of the subject P based on the sensor information 310 and takes statistics of the feature amounts. Then, the leg feature amount calculation unit 152 assumes the result of the statistics of various feature amounts as the leg feature amount.

The relationship calculation unit 153 calculates a correlation coefficient and a regression coefficient (inclination of regression line) indicating relationship between the leg feature amounts or between the upper body feature amounts.

In the present embodiment, for example, in a case where the relationship calculation unit 153 calculates the correlation coefficient and the regression coefficient indicating the relationship between the upper body feature amounts, the correlation coefficient and the regression coefficient are included in the upper body feature amount. Furthermore, in the present embodiment, for example, in a case where the relationship calculation unit 153 calculates the correlation coefficient and the regression coefficient indicating the relationship between the leg feature amounts, the correlation coefficient and the regression coefficient are included in the leg feature amount.

The wobble determination unit 154 determines whether or not the upper body of the subject P wobbles in the load section. Specifically, in a case where the upper body feature amount in the load section exceeds a predetermined range, the wobble determination unit 154 determines that the upper body has wobbled.

The region relationship calculation unit 155 makes the leg feature amount calculation unit 152 calculate the leg feature amount in a predetermined section including time when the upper body has wobbled.

Next, the critical point database creation unit 137 according to the present embodiment will be described. The critical point database creation unit 137 includes a feature amount statistics unit 161 and a load critical point setting unit 162.

Note that to the critical point database creation unit 137 according to the present embodiment, sensor information in a case where the concussion patient group is made to walk for a long time and sensor information in a case where the healthy person group is made to walk for a long time are given in advance. Furthermore, the long time may be time sufficient to detect the critical point on the physical load of the concussion patient.

The feature amount statistics unit 161 according to the present embodiment makes the feature amount calculation unit 135 calculate a feature amount corresponding to a change with time in the value of the acquisition item in the load critical point database 110 for each subject from the sensor information of the concussion patient group and the sensor information of the healthy person group. Then, the feature amount statistics unit 161 takes statistics of the feature amount in each predetermined section for each acquisition item. In other words, the feature amount statistics unit 161 makes the feature amount calculation unit 135 calculate the upper body feature amount and the leg feature amount in each predetermined section for each acquisition item.

The load critical point setting unit 162 assumes the value of the acquisition item of which the value of the concussion patient group is significantly different from the value of the healthy person group as a critical point (threshold) based on the upper body feature amount and the leg feature amount acquired by the feature amount statistics unit 161 for each item indicated in the acquisition items.

Then, the critical point database creation unit 137 creates the load critical point database 110 in which the acquisition item is associated with the critical point (threshold).

Next, the model database creation unit 138 will be described. The model database creation unit 138 according to the present embodiment includes a mean vector calculation unit 171 and a covariance matrix calculation unit 172.

Note that to the model database creation unit 138 according to the present embodiment, sensor information in a case where the concussion patient group is made to walk for a long time and sensor information in a case where the healthy person group is made to walk for a long time are given in advance.

The mean vector calculation unit 171 according to the present embodiment extracts a load section from the sensor information of the concussion patient group and the sensor information of the healthy person group, and in addition, acquires the upper body feature amount and the leg feature amount in the section specified by the section specification unit 134 in the load section for each subject. In other words, the mean vector calculation unit 171 makes the feature amount calculation unit 135 calculate the upper body feature amount and the leg feature amount in the section specified by the section specification unit 134 in the load section for each subject.

Then, the mean vector calculation unit 171 calculates a mean vector indicating a set of the average values of the upper body feature amounts and the leg feature amounts of the plurality of subjects for each of the concussion patient group and the healthy person group.

The covariance matrix calculation unit 172 according to the present embodiment calculates a covariance matrix from the mean vector for each of the concussion patient group and the healthy person group.

Then, the model database creation unit 138 stores the mean vector and the covariance matrix in association with each other for each of the concussion patient group and the healthy person group as the model database 120.

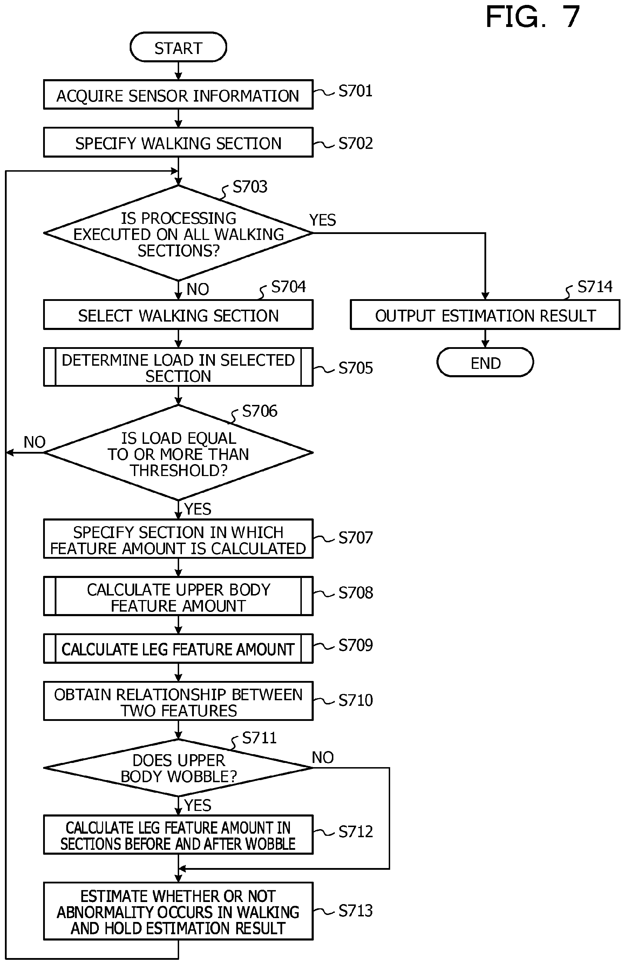

Next, an operation of the abnormal walking estimation processing unit 130 according to the present embodiment will be described with reference to FIG. 7. FIG. 7 is a flowchart for explaining processing of the abnormal walking estimation processing unit according to the first embodiment.

The abnormal walking estimation processing unit 130 according to the present embodiment acquires the sensor information 210 from the sensor 200 and the sensor information 310 from the sensor 300 by the sensor information acquisition unit 131 (step S701).

Subsequently, the abnormal walking estimation processing unit 130 specifies a walking section indicating a section in which the subject P is walking based on the sensor information 310 by the walking section specification unit 132 (step S702). More specifically, the walking section specification unit 132 specifies a start point and an end point of the walking section in the sensor information 310 and specifies a section from the start point to the end point as a single walking section.

Subsequently, the abnormal walking estimation processing unit 130 determines whether or not the processing in and after step S703 is executed on all the walking sections specified by the walking section specification unit 132 by the abnormal walking estimation unit 136 (step S703).

In a case where the processing is executed on all the walking sections in step S703, the procedure proceeds to step S714 described later.

In a case where the processing is not executed on all the walking sections in step S703, the abnormal walking estimation processing unit 130 selects the walking section extracted by the load section extraction unit 133 (step S704).

Subsequently, the load section extraction unit 133 refers to the load critical point database 110 and compares the physical load on the subject P and the threshold of the load critical point database 110 in the selected walking section (step S705). The processing in step S705 will be described later in detail.

Subsequently, the load section extraction unit 133 determines whether or not the physical load on the subject P is equal to or more than the threshold from the comparison result (step S706).

In a case where the load is less than the critical point in step S706, the abnormal walking estimation processing unit 130 returns to step S703.

In a case where the load is equal to or more than the critical point in step S706, the walking section is extracted as a load section, and the procedure proceeds to step S707 described later.

Subsequently, the abnormal walking estimation processing unit 130 specifies a section in which the upper body feature amount and the leg feature amount are calculated in the extracted load section by the section specification unit 134 (step S707).

Subsequently, the abnormal walking estimation processing unit 130 calculates the upper body feature amount of the subject P in the specified section by the upper body feature amount calculation unit 151 of the feature amount calculation unit 135 (step S708). Step S708 will be described later in detail.

Subsequently, the abnormal walking estimation processing unit 130 calculates the leg feature amount in the specified section by the leg feature amount calculation unit 152 of the feature amount calculation unit 135 (step S709). Step S709 will be described later in detail.

Subsequently, the feature amount calculation unit 135 calculates the relationship between the upper body feature amounts and the relationship between the leg feature amounts by the relationship calculation unit 153 (step S710).

Subsequently, the feature amount calculation unit 135 determines whether or not the upper body of the subject P wobbles in the selected walking section from the upper body feature amount by the wobble determination unit 154 (step S711). In a case where the subject P does not wobble in step S711, the abnormal walking estimation processing unit 130 proceeds the procedure to step S713 described later.

In a case where the subject P wobbles in step S711, the feature amount calculation unit 135 calculates the leg feature amount in a predetermined period before and after the wobble occurrence time including the time when the subject P wobbles by the region relationship calculation unit 155 (step S712).

Subsequently, the abnormal walking estimation processing unit 130 estimates whether or not an abnormality occurs in the walking state of the subject P by using the upper body feature amount and the leg feature amount by the abnormal walking estimation unit 136 and holds the result (step S713), and the procedure returns to step S703.

In step S703, in a case where the processing in and after step S703 is executed on all the walking sections, the estimation result output unit 139 outputs the estimation result information (step S714), and the processing is terminated.

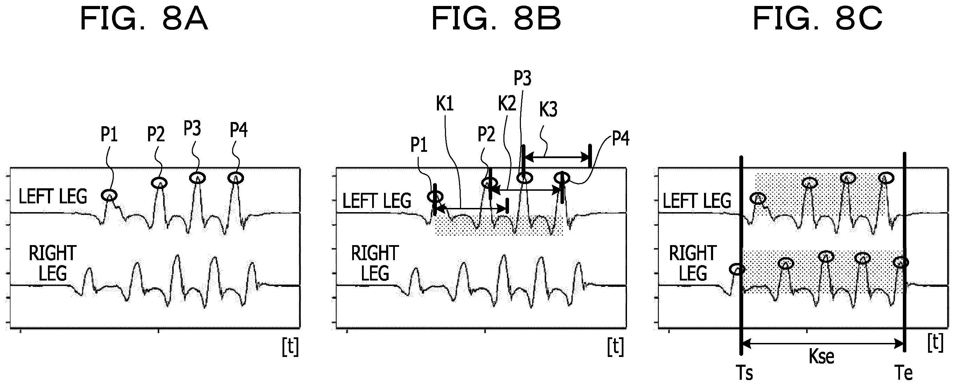

Next, processing in each step in FIG. 7 will be described. First, the processing of the walking section specification unit 132 in step S702 in FIG. 7 will be described with reference to FIG. 8. FIG. 8 is a diagram for explaining specification of the walking section by the walking section specification unit according to the first embodiment.

FIG. 8 illustrates the sensor information 310 in the front-back direction. Generally, in a case where a person is walking, a peak occurs in the sensor information 310 of the sensor attached to the leg of which the toe is separated from the ground and swung forward. The walking section specification unit 132 according to the present embodiment focuses attention on this point and specifies the walking section based on the sensor information 310.

FIGs. 8(A) to 8(C) illustrate the specification of the walking section based on the sensor information 310. Graphs in FIGs. 8(A) to 8(C) illustrate the sensor information 310. The horizontal axis indicates time, and the vertical axis indicates an angular speed.

In a case where the subject P is walking, peaks periodically appear for each foot. In FIG. 8(A), it is found that peaks such as peaks P1, P2, P3, and P4 periodically appear in the sensor information 310 acquired from the sensor 300 of the left leg.

The walking section specification unit 132 according to the present embodiment extracts a section in which the peaks periodically appear and a period between the peaks is within a certain range as a walking section. In FIG. 8(B), in the sensor information 310 of the left leg, it is assumed that a period K1 between the peak P1 and the peak P2, a period K2 between the peak P2 and the peak P3, and a period K3 between the peak P3 and the peak P4 be within a certain range.

In this case, the walking section specification unit 132 specifies the start time and the end time of walking section after detecting that the period between the peaks is similarly within a certain range in the sensor information 310 acquired from the sensor 300 of the right leg, as illustrated in FIG. 8(C). In the present embodiment, for example, in the sensor information 310 of both legs, it is possible to set a time Ts when the first peak appears from among a group of peaks that periodically appear as a start time of walking and a time Te when the final peak appears as an end time of walking. In this case, the walking section is a section Kse from the time Ts to the time Te.

The walking section specification unit 132 according to the present embodiment specifies the walking section from the sensor information 310 as described above.

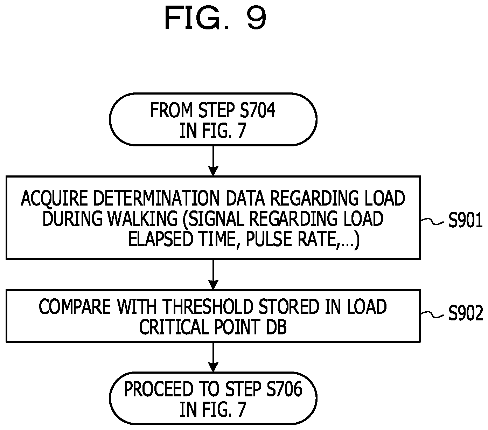

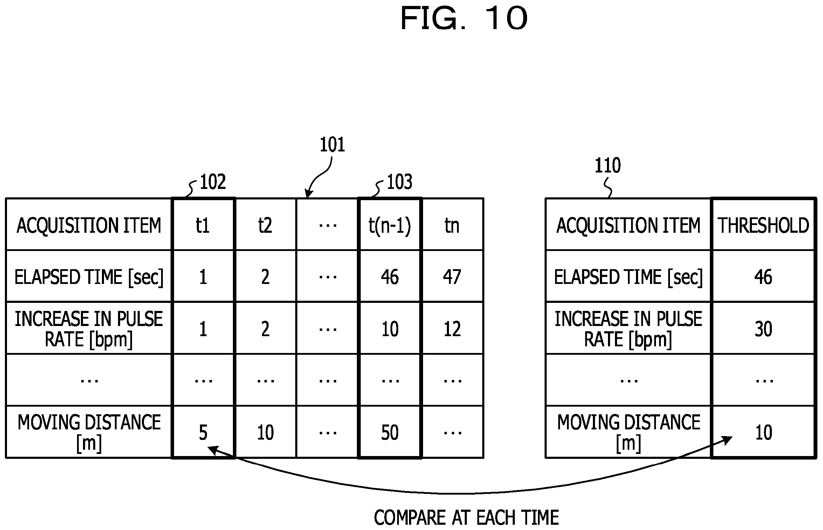

Next, the processing of the load section extraction unit 133 in step S705 in FIG. 7 will be described with reference to FIGs. 9 and 10. FIG. 9 is a flowchart for explaining the processing of the load section extraction unit according to the first embodiment.

The load section extraction unit 133 according to the present embodiment acquires the determination data from the sensor information 210 and 310 by the determination data calculation unit 141 when the walking section is selected in step S704 in FIG. 7 (step S901).

Specifically, the determination data calculation unit 141 acquires the value of each acquisition item in the load critical point database 110 from the sensor information 210 and 310 and sets data including these values as the determination data. The acquisition item is, for example, the elapsed time, the increase in the pulse, the moving distance, and the like.

Subsequently, the load section extraction unit 133 compares the value for each acquisition item included in the determination data acquired in step S901 and the threshold corresponding to the acquisition item in the load critical point database 110 by the critical point determination unit 142 (step S902), and the procedure proceeds to step S706 in FIG. 7.

Here, the determination data made by the determination data calculation unit 141 will be described with reference to FIG. 10. FIG. 10 is a diagram for explaining exemplary determination data according to the first embodiment.

In the present embodiment, the determination data calculation unit 141, for example, acquires a value of the acquisition item for each $t_i$ ($i = 1, 2, 3,..., n$) seconds from the start of the walking section Kse.

In determination data 101 illustrated in FIG. 10, the elapsed time, the increase in the pulse rate, the moving distance, and the like in a case of i = 1, that is, after t1 seconds from the start of the walking section Kse are calculated. Note that the acquisition item may include various items other than the elapsed time, the increase in the pulse rate, and the moving distance. In the present embodiment, a set of the values of the acquisition items at ti seconds in the walking section Kse is the determination data. In the following description, the determination data is referred to as a determination vector. The determination vector according to the present embodiment is acquired for each ti (i = 1, 2, 3,..., n) seconds. That is, the determination vector according to the present embodiment is acquired in time series.

In the determination data 101 illustrated in FIG. 10, a determination vector 102 at the time of t1 seconds in the walking section Kse is (1, 1,..., 5).

In the present embodiment, when the load section extraction unit 133 calculates the determination vector at each time ti by the determination data calculation unit 141, the load section extraction unit 133 compares the determination vector for each ti and the threshold of the load critical point database 110 by the critical point determination unit 142.

Then, in a case where any one of the values of the acquisition items included in the determination vector for each ti exceeds the threshold stored in the load critical point database 110, the critical point determination unit 142 extracts the walking section Kse as the load section. In other words, in a case where any elements of the determination vector for each time in the walking section Kse is equal to or more than the threshold, the critical point determination unit 142 extracts the walking section Kse as the load section.

In the example in FIG. 10, a value of the acquisition item "elapsed time" in a determination vector 103 at a time t (n - 1) is "46[sec]". This value is equal to the threshold corresponding to the acquisition item "elapsed time" in the load critical point database 110. Therefore, the critical point determination unit 142 determines that the physical load on the subject P exceeds the critical point in the walking section Kse and extracts the walking section Kse as a load section.

In the present embodiment, as described above, the load section in which a concussion patient tends to be unbalanced is extracted from the walking section.

Furthermore, it is possible to change the threshold of the acquisition item according to a state before the walking section calculated by using the sensor information 210 and/or the sensor information 310 and compare the threshold with the determination vector. For example, in a case where the state before the walking section is a "long-time sitting state", the acquired threshold is multiplied by a predetermined value (for example, 0.5), and then, may be compared with the determination vector.

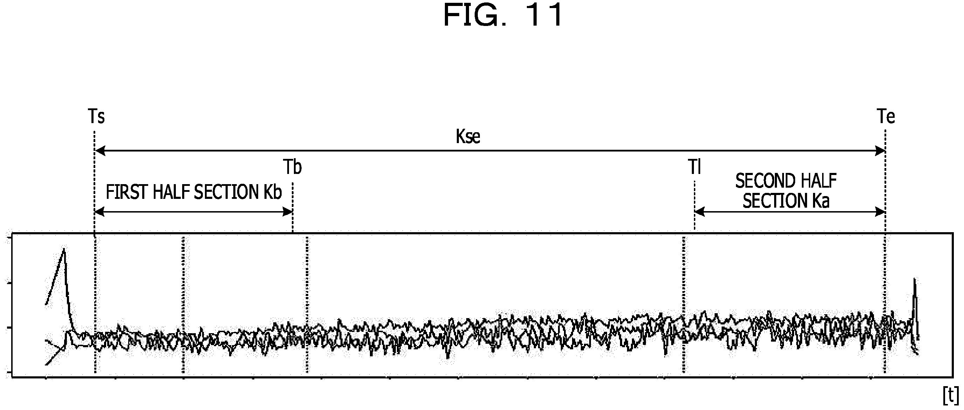

Next, the processing of the section specification unit 134 in step S707 in FIG. 7 will be described with reference to FIG. 11. FIG. 11 is a diagram for explaining specification of the section by the section specification unit according to the first embodiment.

FIG. 11 illustrates an acceleration signal included in the sensor information 210 in each of the front-back direction, the lateral direction, and the vertical direction in the walking section Kse. The vertical axis indicates an acceleration, and the horizontal axis indicates time.

In the example in FIG. 11, a case is illustrated where an element of a determination vector at a time Tl exceeds a threshold in the walking section Kse from the time Ts that is the start time to the time Te that is the end time.

In this case, the section specification unit 134 according to the present embodiment may specify a section Ka from the time Tl to the time Te as a section in which the upper body feature amount and the leg feature amount are calculated by the feature amount calculation unit 135.

In the following description, the section Ka from the time when the element of the determination vector exceeds the threshold to the end time Te of the walking section Kse is referred to as a section half section Ka. Furthermore, in the following description, the time Tl at which the element of the determination vector exceeds the threshold is referred to as a critical point time when the physical load on the subject P reaches the critical point.

Note that the section specification unit 134 according to the present embodiment may, for example, specify the entire walking section Kse as a target section in which the upper body feature amount and the leg feature amount are calculated. Furthermore, the section specification unit 134 according to the present embodiment may specify, for example, a section Kb from the time Ts that is the start time of the walking section Kse to a time Tb predetermined seconds after the time Ts as the target section in which the upper body feature amount and the leg feature amount are calculated. Note that in this case, a user of the information processing apparatus 100 may arbitrarily set the predetermined seconds. In the following description, the section Kb is referred to as a first half section Kb.

Furthermore, in the present embodiment, the first half section Kb and the section half section Ka may be specified as the sections in which the upper body feature amount and the leg feature amount are calculated, and all of the walking section Kse, the first half section Kb, and the section half section Ka may be specified as the sections in which the upper body feature amount and the leg feature amount are calculated.

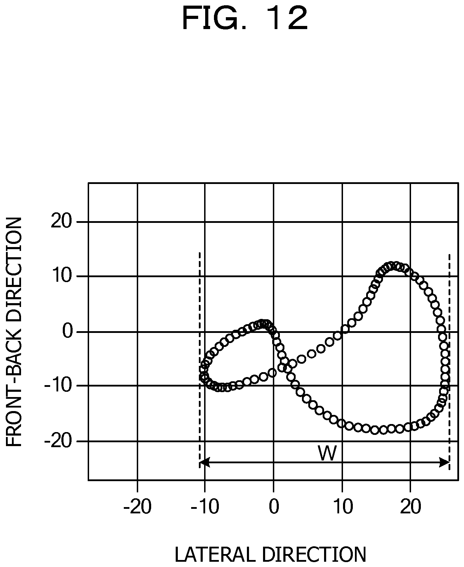

Next, the processing of the feature amount calculation unit 135 in step S708 in FIG. 7 will be described with reference to FIGs. 12 to 14.

The feature amount calculation unit 135 according to the present embodiment calculates the upper body feature amount indicating the motion of the upper body of the subject P by the upper body feature amount calculation unit 151.

More specifically, the upper body feature amount calculation unit 151 acquires the moving width and the angle of the waist each time when the subject P walks for two steps and calculates an average value and a standard deviation of the acquired moving width of the waist as the upper body feature amount. Note that in the present embodiment, the maximum value and the minimum value of the moving width of the waist may be included in the upper body feature amount.

Furthermore, in the present embodiment, for each of the moving width and the angle of the waist, an arithmetic average and a median may be used instead of the average value. Moreover, in the present embodiment, for each of the moving width and the angle of the waist, a variation coefficient may be used instead of the standard deviation.

First, calculation of the moving width of the waist by the upper body feature amount calculation unit 151 will be described.

FIG. 12 is a diagram for explaining the processing of the feature amount calculation unit according to the first embodiment. In FIG. 12, calculation of the moving width of the waist will be described.

In the present embodiment, a movement of the waist in the vertical direction in the walking section Kse is considered as a landing motion caused by walking. Then, in the present embodiment, a distance from the position of the motion of the waist in the vertical direction to a position of the next motion in the vertical direction is assumed as a single step, and a locus of the waist for each two steps of the subject P is calculated.

In FIG. 12, the locus of the waist of the subject P for each two steps is indicated by points indicated by the coordinates of the sensor information 210 in the front-back direction and the coordinates in the lateral direction.

The upper body feature amount calculation unit 151 according to the present embodiment calculates a moving width W of the locus of the waist in the lateral direction for each two steps while the subject P is walking in the section specified by the section specification unit 134 as illustrated in FIG. 12. The upper body feature amount calculation unit 151 according to the present embodiment similarly calculates the moving widths of the waist in the vertical direction and the front-back direction.

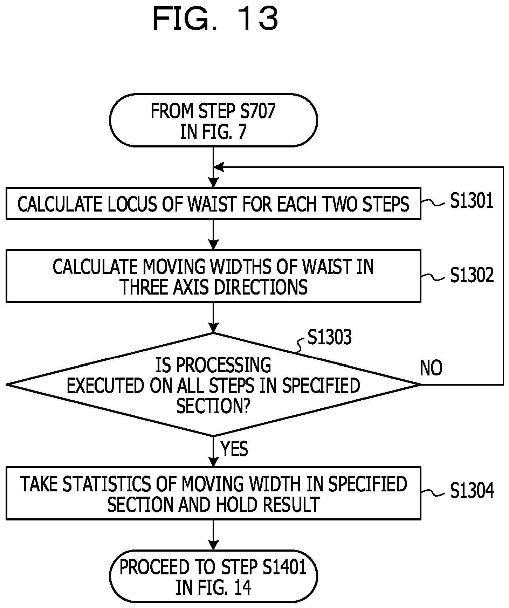

FIG. 13 is a flowchart for explaining the processing for calculating the moving width of the waist by the upper body feature amount calculation unit according to the first embodiment.

When the section specification unit 134 specifies the section in which the upper body feature amount is calculated, the upper body feature amount calculation unit 151 according to the present embodiment calculates the locus of the waist of the subject P for each two steps in the specified section (step S1301). Subsequently, the upper body feature amount calculation unit 151 calculates moving widths of the waist in the three axis directions from the calculated locus (step S1302). The three axis directions include the lateral direction, the vertical direction, and the front-back direction.

Subsequently, the upper body feature amount calculation unit 151 determines whether or not the processing in steps S1301 and S1302 is executed on all the steps (one step) in the section specified by the section specification unit 134 (step S1303). In a case where the processing is not executed on all the steps in step S1303, the upper body feature amount calculation unit 151 returns to step S1301.

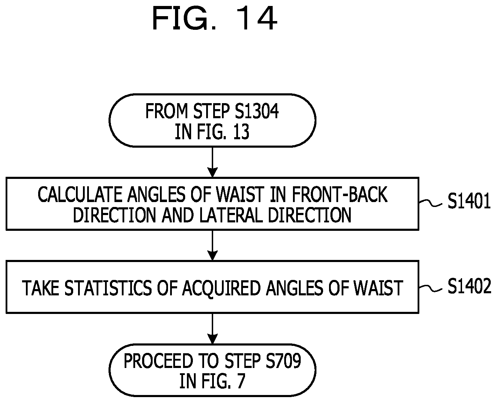

In a case where the processing is executed on all the steps in step S1303, the upper body feature amount calculation unit 151 takes statistics of the calculated moving width of the waist for each two steps and holds the result as the upper body feature amount (step S1304). Then, the procedure proceeds to step S1401 in FIG. 14 described later.

Note that in step S1304, to take the statistics of the moving width of the waist is specifically, to calculate the average value and the standard deviation of the moving width of the waist, to extract the maximum value and the minimum value, and the like.

Furthermore, in the present embodiment, in a case where the section specification unit 134 specifies, for example, the first half section Kb and the section half section Ka, a ratio indicating a relation between the upper body feature amount in the first half section Kb and the upper body feature amount in the section half section Ka may be included in the upper body feature amount.

Next, calculation of the angle of the waist by the upper body feature amount calculation unit 151 will be described.

The upper body feature amount calculation unit 151 according to the present embodiment calculates an angle of inclination with respect to a gravitational acceleration of the sensor 200 relative to the front-back direction and an angle of inclination with respect to a gravitational acceleration of the sensor 200 with respect to the lateral direction from the sensor information 210 as the angle of the waist.

In the following description, the angle of inclination relative to the gravitational acceleration of the sensor 200 with respect to the front-back direction is referred to as an angle of the waist in the front-back direction, and the angle of inclination relative to the acceleration of the sensor 200 with respect to the lateral direction is referred to as an angle of the waist in the lateral direction.

The angle of the waist in the front-back direction and the angle of the waist in the lateral direction are calculated according to the following formula (1) .

[Expression 1]

$$\text{angle of waist in front-back direction: } \varphi_{ap} = \tan^{-1}\left(\frac{-Z}{\sqrt{X^2 + Y^2}}\right)$$

$$\text{angle of waist in lateral direction: } \varphi_{ml} = \tan^{-1}\left(\frac{X}{Y}\right)$$

$$\text{... formula (1)}$$

Note that, here, the references X, Y, and Z are data respectively indicating an acceleration in the X-axis direction, an acceleration in the Y-axis direction, and an acceleration in the Z-axis direction in time series acquired from the sensor information 210, and X = (X1, X2,..., Xn), Y = (Y1, Y2,..., Yn), and Z = (Z1, Z2,..., Zn) are satisfied.

FIG. 14 is a flowchart for explaining processing for calculating the angle of the waist by the upper body feature amount calculation unit according to the first embodiment.

The upper body feature amount calculation unit 151 according to the present embodiment calculates angles of the waist in the front-back direction and the lateral direction at all the times from the sensor information 210 acquired in the section specified by the section specification unit 134 based on the formula (1) (step S1401) .

Subsequently, the upper body feature amount calculation unit 151 takes statistics for each of the angle of the waist in the front-back direction and the angle of the waist in the lateral direction acquired in step S1401 and holds the result as the upper body feature amount (step S1402). Then, the procedure proceeds to step S709 in FIG. 7.

Note that to take the statistics of the angle of the waist in step S1402 is specifically, to calculate an average value and a standard deviation of the angle of the waist, to extract the maximum value and the minimum value, and the like.

That is, the upper body feature amount according to the present embodiment includes at least the average value and the standard deviation of the moving widths of the waist in the three axis directions and the average value and the standard deviation of the angles of the waist in the front-back direction and the lateral direction.

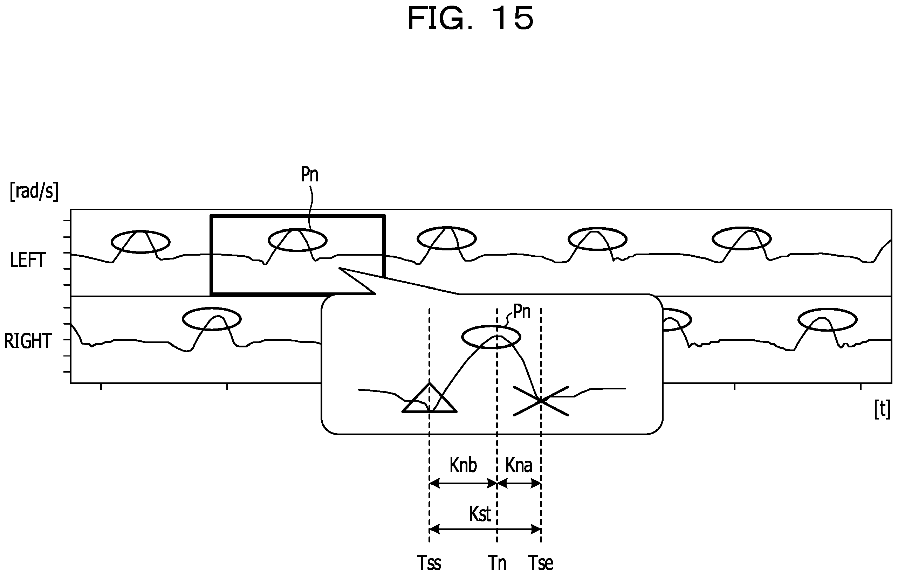

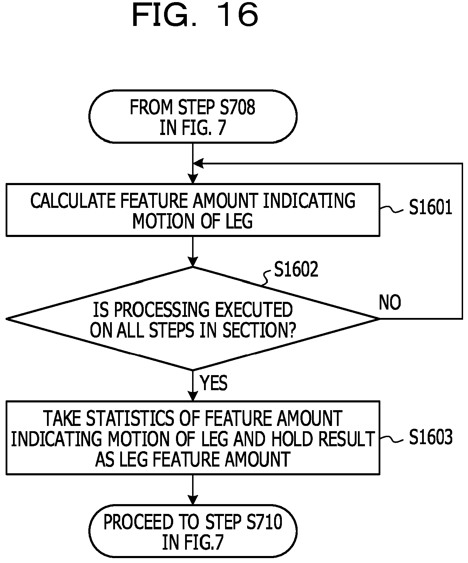

Next, the processing of the feature amount calculation unit 135 in step S709 in FIG. 7 will be described with reference to FIGs. 15 and 16.

The feature amount calculation unit 135 according to the present embodiment calculates the leg feature amount indicating the motions of both legs of the subject P by the leg feature amount calculation unit 152.

First, the motions of the legs of the subject P in the present embodiment will be described with reference to FIG. 15. FIG. 15 is a diagram for explaining the motions of the legs of the subject according to the first embodiment.

FIG. 15 illustrates the sensor information 310 acquired from the sensors 300 respectively attached the left and the right legs. The vertical axis indicates an angular speed, and the horizontal axis indicates time.

In the walking section, the peaks periodically appear in the sensor information 310 of both legs. In FIG. 15, a single step (step) in the walking of the subject P will be described by using a peak Pn in the sensor information 310 of the left leg.

In the present embodiment, times when the value of the sensor information 310 (value of angular speed) is minimized before and after a time Tn when the peak Pn appears are assumed as a start time Tss and an end time Tse. The start time Tss of the step indicates a timing when the toe of the leg of the subject P is separated from the ground or the floor. The end time Tse of the step indicates a timing when the heel of the subject P has contact with the ground.

Therefore, in the example in FIG. 15, each of a section Knb from the start time Tss to the time Tn when the peak is detected and a section Kst, including a section Kna, from the time Tn to the end time Tse is the single step of the subject P. In other words, the single step indicates a motion from the time when the toe of one of the legs of the subject P is separated from the ground to the time when the heel has contact with the ground.

Based on the above motion, the leg feature amount calculation unit 152 according to the present embodiment calculates moving widths of the length to the left and right, a standing time, a leg raising width, a walking speed, a leg raising width while idling the leg, a minimum rotation speed of the coronal plane, a time to the middle of leg idling, and a length of stride, and assumes the results of the statistics of the calculation results as the leg feature amounts.

The moving width of the leg to the left or right indicates an integral value of the angular speed in the lateral direction in the section Kst from the start time Tss of the step to the end time Tse. Note that in the present embodiment, the moving width of the leg to the left or right may be a value obtained by constant multiplying the integral value.

The standing time indicates a time interval from an end time Tse of an Nth step to a start time Tss of an N+1th step.

The leg raising width indicates an integral value of an angular speed in the vertical direction in the section Kst from the start time Tss of the step to the end time Tse. Note that, in the present embodiment, the leg raising width may be a value obtained by constant multiplying the integral value.

The walking speed indicates an angular speed at the time Tn. Note that in the present embodiment, the walking speed may be a value obtained by constant multiplying the angular speed.

The leg raising width while idling the leg indicates an integral value of each acceleration in the vertical direction in the section Kst from the start time Tss of the step to the time Tn. Note that the leg raising width while idling the leg may be a value obtained by constant multiplying the integral value.

The minimum rotation speed of the coronal plane indicates the minimum value of the angular speed in the vertical direction in the section Kst from the start time Tss of the step to the end time Tse.

The time to the middle of leg idling indicates the section Knb from the start time Tss of the step to the time Tn.

The length of stride indicates a moving distance from the start time Tss of the step to the end time Tse.

Hereinafter, the calculation of the leg feature amount by the leg feature amount calculation unit 152 according to the present embodiment will be described with reference to FIG. 16. FIG. 16 is a flowchart for explaining processing for calculating the leg feature amount by the leg feature amount calculation unit according to the first embodiment.

When completing the calculation of the upper body feature amount by the upper body feature amount calculation unit 151 in step S708, the leg feature amount calculation unit 152 according to the present embodiment calculates a value indicating the motion of the leg of the subject P for each step in the section specified by the section specification unit 134 (step S1601). Specifically, the leg feature amount calculation unit 152 calculates the eight kinds of values described above.

Subsequently, the leg feature amount calculation unit 152 determines whether or not the eight kinds of values are calculated for all the steps in the section specified by the section specification unit 134 (step S1602). In step S1602, in a case where the feature amounts for all the steps are not calculated, the leg feature amount calculation unit 152 returns to step S1601.

In a case where the eight kinds of values for all the steps are calculated in step S1602, the leg feature amount calculation unit 152 holds the result of the statistics of the calculated values as the leg feature amount (step S1603), and the procedure proceeds to step S710 in FIG. 7.

Specifically, the leg feature amount calculation unit 152 may use, for example, values obtained by calculating the average value and the standard deviation of each of the eight kinds of values as the leg feature amounts. In this case, the leg feature amount includes eight average values and standard deviations. Furthermore, the leg feature amount calculation unit 152 may acquire the minimum value and the maximum value of each of the eight feature amounts as the leg feature amount. Furthermore, the leg feature amount calculation unit 152 according to the present embodiment may use the feature amount acquired based on the left-right symmetry of the waveform of the sensor information 310 as the leg feature amount. In other words, the leg feature amount calculation unit 152 may use not only the average value of the walking speed of the left leg and the average value of the walking speed of the right left leg but also a difference between the average values of the walking speeds of the left and right legs (example of left-right symmetry).

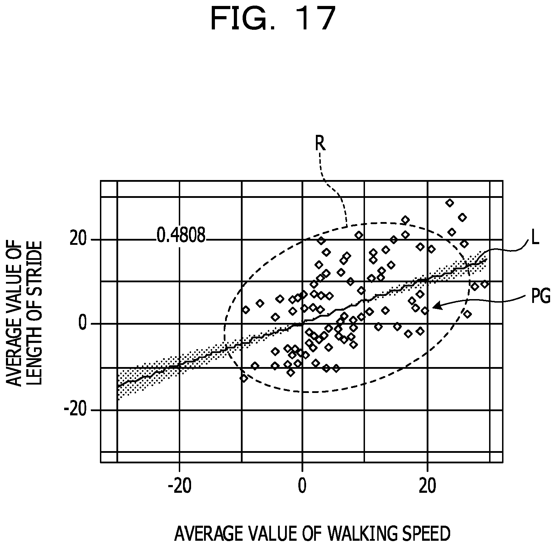

Next, the processing of the relationship calculation unit 153 in step S710 in FIG. 7 will be described with reference to FIG. 17.

The relationship calculation unit 153 according to the present embodiment calculates a relationship between the upper body feature amounts calculated by the upper body feature amount calculation unit 151 and a relationship between the leg feature amounts calculated by the leg feature amount calculation unit 152.

FIG. 17 is a diagram for explaining calculation of the regression coefficient by the relationship calculation unit according to the first embodiment. FIG. 17 illustrates an example in which a regression coefficient indicating a relationship between the average value of the length of stride and the average value of the walking speed included in the leg feature amount is calculated.

In FIG. 17, the vertical axis indicates the average value of the length of stride, and the horizontal axis indicates the average value of the walking speed. In the example in FIG. 17, a point group PG indicated by the average value of the walking speed and the average value of the length of stride obtained from the leg feature amount of the subject P exists in a region R. Then, in FIG. 17, it is found that a regression line L and a regression coefficient = 0.4808 are calculated from the point group PG. In the present embodiment, the regression coefficient = 0.4808 is included in the leg feature amount.

Furthermore, in FIG. 17, an example is described in which the relationship between the average value of the walking speed and the average value of the length of stride is calculated. However, the present invention is not limited to this.

The relationship calculation unit 153 according to the present embodiment may, for example, calculate a regression coefficient indicating a relationship between a standard deviation of the walking speed and a standard deviation of the length of stride and include the regression coefficient as the leg feature amount.

In addition, the relationship calculation unit 153 according to the present embodiment may calculate a regression coefficient indicating a relationship between the average value of the moving widths of the waist in the three axis directions and the average value of the angle of the waist in the front-back direction included in the upper body feature amount and include the regression coefficient as the upper body feature amount. Furthermore, the relationship calculation unit 153 may calculate a regression coefficient indicating a relationship between a standard deviation of the moving width of the waist in each of the three axis directions and a standard deviation of the angle of the waist in the front-back direction, a regression coefficient indicating a relationship between an average value of the moving width of the waist in each of the three axis directions and an average value of the angle of the waist in the lateral direction, and a regression coefficient indicating a relationship between the standard deviation of the moving width of the waist in each of the three axis directions and the standard of the angle of the waist in the lateral direction and may include the calculated regression coefficients as the upper body feature amount.

The relationship calculation unit 153 according to the present embodiment may calculate a regression coefficient and a correlation coefficient indicating a relation between all the combinations that may be made by using values included in the leg feature amounts and all the combinations that may be made by using values included in the upper body feature amounts and may include the coefficients in the leg feature amount and the upper body feature amount.

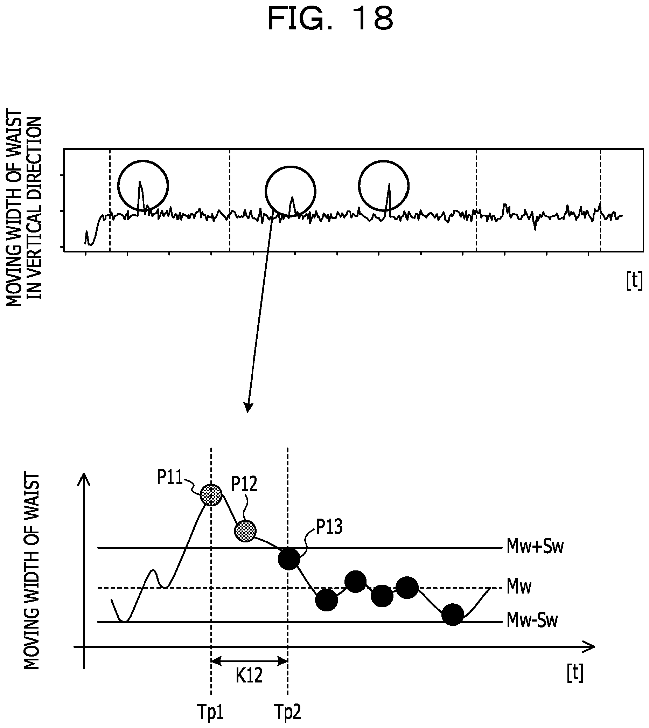

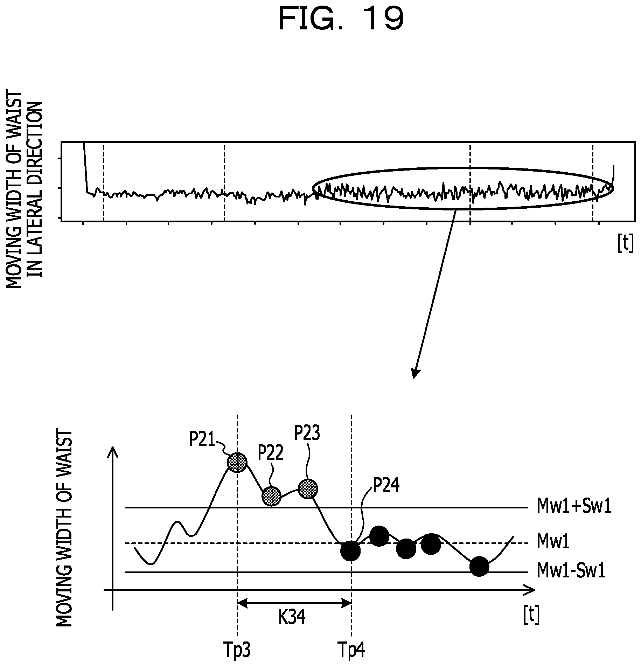

Next, the processing of the wobble determination unit 154 in step S711 in FIG. 7 will be described with reference to FIGs. 18 and 19.

In the feature amount calculation unit 135 according to the present embodiment, the wobble determination unit 154 determines whether or not the upper body of the subject P wobbles based on the upper body feature amount.

In a case where the moving width of the waist in the vertical direction included in the upper body feature amount exceeds a predetermined range, the wobble determination unit 154 according to the present embodiment determines that the subject P wobbles. Note that in the example in FIG. 18, the moving width in the vertical direction is used. However, the direction of the moving width is not limited to this. The direction of the moving width is not limited to the vertical direction, and the moving widths in the lateral direction and the front-back direction may be used.

Furthermore, in a case where it is determined that the subject P wobbles, the wobble determination unit 154 according to the present embodiment determines a kind of the wobble.

FIG. 18 is a first diagram for explaining the determination of the wobble by the wobble determination unit according to the first embodiment. FIG. 18 illustrates a case where the subject P temporarily loses one's balance.

When the value of the moving width of the waist in the vertical direction exceeds a predetermined range, the wobble determination unit 154 according to the present embodiment determines that the subject P wobbles.

The predetermined range in FIG. 18 is from Mw - Sw to Mw + Sw in a case where it is assumed that the average value of the moving width of the waist in the vertical direction be Mw and the standard deviation of the waist in the vertical direction be Sw. That is, in a case where the value of the moving width of the waist in the vertical direction < Mw - Sw or in a case where Mw + Sw < the value of the moving width of the waist in the vertical direction, the wobble determination unit 154 determines that the subject P wobbles.

In the example in FIG. 18, the value of the moving width of the waist in the vertical direction is a value P11 at a time Tp1 and exceeds Mw + Sw. Therefore, the wobble determination unit 154 determines that the subject P wobbles.

Furthermore, in the example in FIG. 18, after the value P11 exceeds the predetermined range at the time Tp1, a value P12 exceeds the predetermined range. Then, in the example in FIG. 18, a value P13 of the waist returns to a value within the predetermined range at a time Tp2. That is, in the example in FIG. 18, a section from a time when it is determined that the wobble occurs at the time Tp1 to the time when the value of the moving width of the waist in the vertical direction returns to the value within the predetermined range is a section K12.

In the example in FIG. 18, it is assumed that the length of the section K12 be equal to or less than a predetermined value. In this case, the value of the moving width of the waist in the vertical direction in the section K12 is determined as a value indicating that the subject P temporarily loses one's balance. Note that the predetermined value in the present embodiment may be, for example, about three seconds and may be shorter than three seconds.

In the following description, the value of the moving width of the waist in the vertical direction indicating that the subject P temporarily loses one's balance is referred to as an instantaneous feature amount. Therefore, in the example in FIG. 18, the values P11 and P12 of the moving width of the waist in the vertical direction are the instantaneous feature amounts.

FIG. 19 is a second diagram for explaining the determination of the wobble by the wobble determination unit according to the first embodiment. FIG. 19 illustrates a case where the subject P wobbles for a while after losing one's balance.

When the value of the moving width of the waist in the lateral direction exceeds the predetermined range, the wobble determination unit 154 according to the present embodiment determines that the subject P wobbles. Note that in the example in FIG. 19, the moving width in the lateral direction is used. However, the direction of the moving width is not limited to this. The direction of the moving width of the waist is not limited to the lateral direction, and the moving width in the front-back direction or the vertical direction may be used.

The predetermined range in FIG. 19 is from Mw1 - Sw1 to Mw1 + Sw1 in a case where it is assumed that the average value of the moving width of the waist in the lateral direction be Mw1 and the standard deviation of the waist in the lateral direction be Sw1. That is, in a case where the value of the moving width of the waist in the lateral direction < Mw1 - Sw1 or in a case where Mw1 + Sw1 < the value of the moving width of the waist in the vertical direction, the wobble determination unit 154 determines that the subject P wobbles.

Furthermore, in the example in FIG. 19, values P22 and P23 after a value P21 of the moving width of the waist in the lateral direction at a time Tp3 exceeds the predetermined range remain outside the predetermined range. Then, in the example in FIG. 19, at a time Tp4, a value P24 of the moving width of the waist in the lateral direction returns to a value within the predetermined range. That is, in the example in FIG. 19, a section from a time when it is determined that the wobble occurs at the time Tp3 to the time when the value of the moving width of the waist in the lateral direction returns to the value within the predetermined range is a section K34.

In the example in FIG. 19, it is assumed that the length of the section K34 is longer than a predetermined value. In that case, the value of the moving width of the waist in the lateral direction in the section K34 is determined as a value indicating that the subject P wobbles for a while after losing one's balance.

In the following description, the value of the moving width of the waist in the lateral direction indicating that the subject P wobbles for a while after losing one's balance is referred to as a continuous feature amount. Therefore, in the example in FIG. 19, the values P21, P22, and P23 of the moving width of the waist in the lateral direction are the continuous feature amounts.

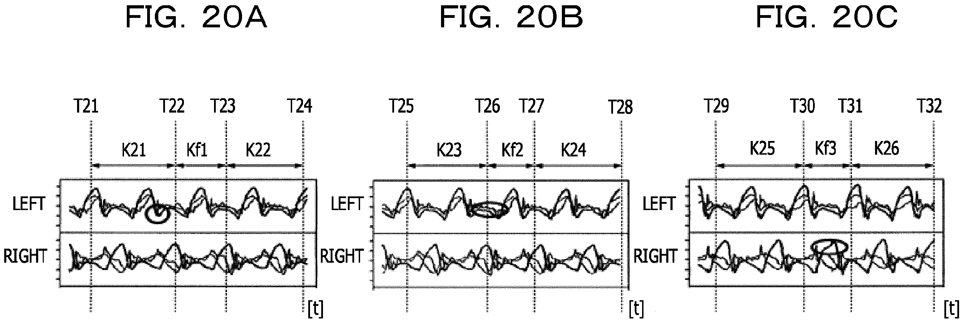

Next, the processing of the region relationship calculation unit 155 in step S712 in FIG. 7 will be described with reference to FIG. 20.

In the present embodiment, attention is focused on a point such that a movement different from the normal movement is included in the movement of the leg of the subject P in the section before and after the wobble in a case where the upper body of the subject P wobbles, and a relationship between the wobble of the upper body and the movements of both legs is obtained.

FIG. 20 is a diagram for explaining the relation between the wobble of the upper body and the movements of both legs. In graphs in FIGs. 20(A), 20(B), and 20(C), the vertical axis indicates the sensor information 310 (angular speed), and the horizontal axis indicates time.

In the example in FIG. 20(A), a case is illustrated where the wobble of the upper body is detected in a section Kf1 from a time T22 to a time T23. In this case, it is found that the heel of the left leg stumbles at the time of grounding in a section K21 before the section Kf1 in which the wobble occurs.

Furthermore, in the example in FIG. 20(B), a case is illustrated where the wobble of the upper body is detected in a section Kf2 from a time T26 to a time T27. In this case, it is found that the movement of the left leg when the right leg is raised is disturbed in a section K25 before the section Kf1 in which the wobble occurs.

Furthermore, in the example in FIG. 20(C), a case is illustrated where the wobble of the upper body is detected in a section Kf3 from a time T30 to a time T31. In this case, it is found that the movement of the right leg is disturbed in the section Kf1 in which the wobble occurs.

Therefore, the region relationship calculation unit 155 according to the present embodiment calculates the feature amount of the leg in a predetermined period including the section in which the upper body wobbles and the sections before and after the section.

In the present embodiment, it is assumed that each of the sections before and after the section in which the wobble occurs be a section of four steps of the subject P (four steps).

Therefore, in FIG. 20(A), the section K21 from the time T21 to the time T22 is set as a section four steps before the section Kf1, and the section K22 from the time T23 to the time T24 is set as a section four steps after the section Kf1. Then, the region relationship calculation unit 155 makes the leg feature amount calculation unit 152 calculate the leg feature amount in the predetermined section from the time T21 to the time T24 including the section K21, the section Kf1, and the section K22.

Similarly, the region relationship calculation unit 155 makes the leg feature amount calculation unit 152 calculate a leg feature amount in a predetermined section from a time T25 to a time T28 including a section K23 from the time T25 to the time T26, the section Kf2, and a section K24 from the time T27 to the time T28.

Similarly, the region relationship calculation unit 155 makes the leg feature amount calculation unit 152 calculates a leg feature amount in a predetermined section from a time T29 to a time T32 including a section K25 from the time T29 to the time T30, the section Kf3, and a section K26 from the time T31 to the time T32.

In the present embodiment, as described above, the region relationship calculation unit 155 makes the leg feature amount calculation unit 152 calculate the leg feature amount in the predetermined section so as to calculate the relationship between the plurality of regions (upper body and both legs).

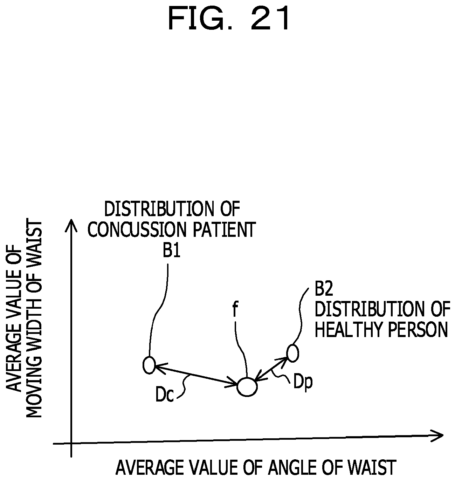

Next, the processing of the abnormal walking estimation unit 136 in step S713 in FIG. 7 will be described with reference to FIG. 21.

The abnormal walking estimation unit 136 according to the present embodiment estimates whether or not an abnormality occurs in the walking state of the subject P based on the upper body feature amount and the leg feature amount calculated by the feature amount calculation unit 135 and the model database 120.

In the following description, for convenience of description, the description will be made as assuming that the upper body feature amount calculated by the feature amount calculation unit 135 be the average value of the moving width of the waist in one axis direction and the average value of the angle of the waist in one axis direction. In this case, the upper body feature amount $f = (f_1, f_2)$ calculated by the feature amount calculation unit 135 is indicated. Here, the

reference $f_1$ indicates the average value of the moving width of the waist in one axis direction, and the reference $f_2$ indicates the average value of the angle of the waist in one axis direction.

The abnormal walking estimation unit 136 according to the present embodiment calculates a Mahalanobis Distance ($D_p$, $D_c$) with each distribution. Note that each distribution includes a distribution of the average value of the moving width of the waist and the angle of the waist indicated by the mean vector and the covariance matrix of the concussion patient group stored in the model database 120 and a distribution of the average value of the moving width of the waist and the angle of the waist indicated by the mean vector and the covariance matrix of the healthy person group.

The Mahalanobis Distance $D_p$ is calculated by the following formula (2).

[Expression 2]

$$D_p = (f - \overline{F}_p)^T A_p^{-1} (f - \overline{F}_p) \qquad \ldots \text{ formula (2)}$$

$\overline{F}_p$, $A_p$ :mean vector and covariance matrix of concussion patient group

Note that in the formula (2), the Mahalanobis Distance $D_c$ indicates a distance between the distribution of the concussion patient group and an upper body feature amount f. The abnormal walking estimation unit 136 calculates the Mahalanobis Distance $D_p$ indicating a distance between the distribution of the healthy person group and the upper body feature amount f by substituting the mean vector and the covariance matrix of the concussion patient group in the formula (2) with the mean vector and the covariance matrix of the healthy person group.

FIG. 21 is a diagram for explaining estimation by the abnormal walking estimation unit according to the first embodiment.

For example, in a case of $D_p < D_c$, the abnormal walking estimation unit 136 according to the present embodiment estimates that the subject P has an abnormality in walking due to the decrease in the balance ability caused by the concussion. In other words, in a case where the upper body feature amount f is closer to a distribution B1 of the concussion patient group than a distribution B2 of the healthy person group, the abnormal walking estimation unit 136 according to the present embodiment estimates that the abnormality occurs in the walking state of the subject P.

Note that in the diagram illustrated in FIG. 21, the number of values of the upper body feature amounts f is two. Therefore, the number of axes is two. However, in actual, corresponding axes as many as the number of the values of the upper body feature amounts and the number of the values of the leg feature amounts exist.

Furthermore, in the above description, it is estimated that the abnormality occurs in walking in a case of $D_p < D_c$. However, the present invention is not limited to this.

A condition to estimate that "an abnormality occurs in walking" may be determined by, for example, a medical worker who uses the information processing apparatus 100 and the like.

For example, in a case where the plurality of pieces of sensor information 210 and 310 acquired in daily life of the subject P is used, the abnormal walking estimation unit 136 may calculate the Mahalanobis Distance ($D_p$, $D_c$) for each of the plurality of pieces of sensor information 210 and 310. Then, the abnormal walking estimation unit 136 may estimate whether or not the abnormality occurs in walking of the subject P from the plurality of Mahalanobis Distances ($D_p$, $D_c$).

Furthermore, in the present embodiment, for example, in a case where $D_p < D_c$ is satisfied and $D_p$ is shorter than a predetermined distance, the value f is closer to the healthy person group. Therefore, the abnormal walking estimation unit 136 may estimate that the subject P is likely to recover.

Furthermore, in a case where a ratio of the load sections in which $D_p < D_c$ is satisfied relative to all the extracted load sections is equal to or more than a predetermined value, the abnormal walking estimation unit 136 according to the present embodiment may estimate that the abnormality occurs in the walking state of the subject P.

Furthermore, the abnormal walking estimation unit 136 according to the present embodiment may, for example, estimate whether or not the subject P tends to recover by calculating a difference with daily records. Specifically, for example, by comparing the current Mahalanobis Distance $D_p$ with the Mahalanobis Distance $D_p$ of the previous day or one week ago, it may be estimated that the subject P is recovered when the Mahalanobis Distance $D_p$ is equal to or less than a half of the value on the previous day or one week ago.

Furthermore, the abnormal walking estimation unit 136 may estimate whether or not the subject P is likely to recover based on the past record of the concussion patient having similar gender and age to the subject P. Specifically, for example, the Mahalanobis Distance $D_p$ on or after the concussion of the concussion patient who has smoothly recovered is recorded in advance. Then, the Mahalanobis Distance $D_p$ of a concussion patient having the gender and the age close to the subject P is extracted. Next, the Mahalanobis Distance $D_p$ of the day after the same days from the occurrence of the concussion is compared with the Mahalanobis Distance $D_p$ of the subject P. If both Mahalanobis Distances are substantially the same, it may be estimated that the subject P is recovered.

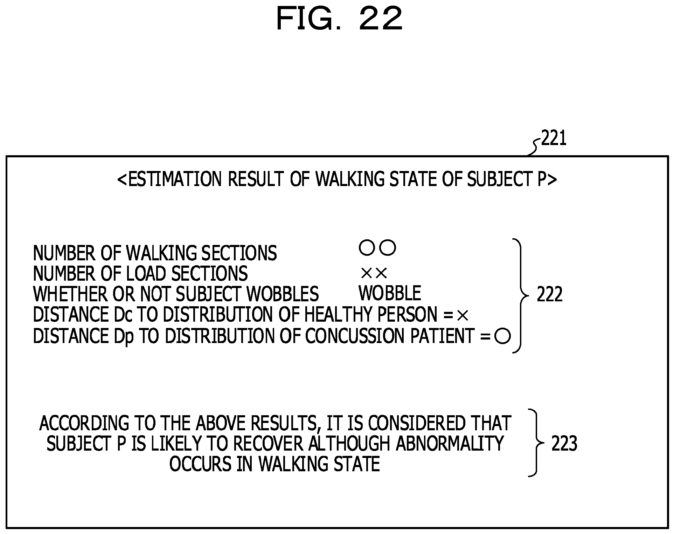

Next, the processing of the estimation result output unit 139 in step S714 in FIG. 7 will be described with reference to FIG. 22. FIG. 22 is a diagram illustrating an exemplary output of estimation result information according to the first embodiment.

In FIG. 22, an example of a screen is illustrated in which the estimation result information is displayed on a display of the information processing apparatus 100 by the estimation result output unit 139.

In a screen 221 illustrated in FIG. 22, information 222 indicating the result of the processing that is the basis of the estimation of the abnormal walking estimation processing unit 130 and information 223 indicating the estimation result are displayed as the estimation result information.

The information 222 includes the number of extracted walking sections, the number of load sections, whether or not the subject P wobbles, the Mahalanobis Distance ($D_p$, $D_c$), and the like as the estimation results of the walking state of the subject P.

Furthermore, the information 223 indicates that the subject P is likely to recover although the abnormality occurs in the walking state of the subject P, from the information 222.

Note that in the example in FIG. 22, the information 222 is displayed. However, the present invention is not limited to this. The estimation result information may include only the information 223. Furthermore, whether or not to display the information 222 may be determined by setting of a user of the information processing apparatus 100.

Moreover, the screen 221 in FIG. 22 is displayed on the display of the information processing apparatus 100. However, the present invention is not limited to this. The screen 221 may be displayed on a display and the like of an external device that can communicate with the information processing apparatus 100. Furthermore, in a case where the sensor 200 is mounted on a terminal device of a smartphone and the like, the screen 221 may be displayed on a display of the terminal device on which the sensor 200 is mounted.

Moreover, in the present embodiment, the information displayed on the screen 221 may be output as a printed matter and may be output as the estimation result information.

Next, creation of the load critical point database 110 and the model database 120 according to the present embodiment will be described. First, the creation of the load critical point database 110 will be described.

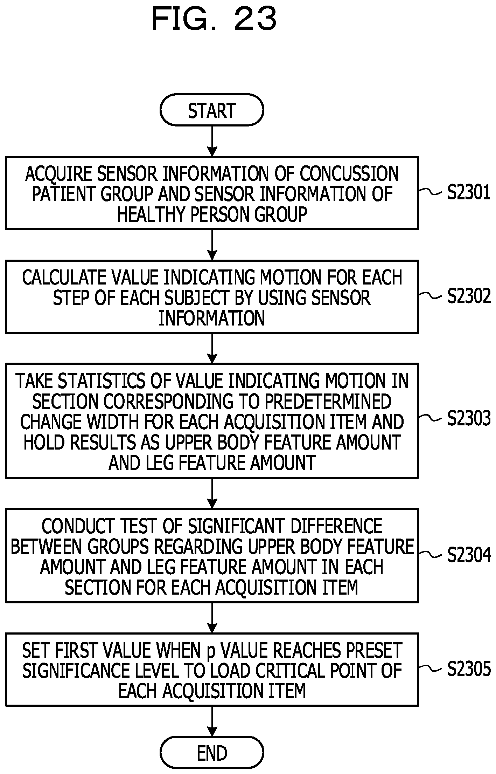

FIG. 23 is a flowchart for explaining the processing of the critical point database creation unit according to the first embodiment.

The critical point database creation unit 137 according to the present embodiment acquires the sensor information of the concussion patient group and the sensor information of the healthy person group by the sensor information acquisition unit 131 (step S2301). Note that these pieces of sensor information correspond to the sensor information 210 and the sensor information 310 and may be information collected from subjects belonging to each group in advance.

Subsequently, the critical point database creation unit 137 calculates a value indicating the motion of the upper body for each step (one step) and a value indicating the motion of both legs based on the sensor information of each subject by the feature amount statistics unit 161 (step S2302). In other words, the feature amount statistics unit 161 calculates the value indicating the motion of the upper body and the value indicating the motion of both legs for each step for each subject.

Note that the value indicating the motion of the upper body includes the moving widths of the waist in the three axis directions and the angles of the waist in the two axis direction, and the value indicating the motion of both legs includes the moving width of the leg to the left and right, the standing time, the leg raising width, the walking speed, the leg raising width while idling the leg, the minimum rotation speed of the coronal plane, the time to the middle of leg idling, and the length of stride.

Subsequently, the feature amount statistics unit 161 assumes the result of the statistics of the values indicating the motions of the upper body as the upper body feature amount in each section corresponding to a predetermined change width for each acquisition item in the load critical point database 110 and holds the result of the statistics of the value indicating the motions of both legs as the leg feature amount (step S2303).

Subsequently, the critical point database creation unit 137 conducts a test of a difference between the average values of the groups, for example, the t-test regarding the upper body feature amount and the leg feature amount of each section for each acquisition item by the load critical point setting unit 162 (step S2304).

Subsequently, the load critical point setting unit 162 acquires a first value at the time when an index used to determine whether or not the acquisition item is statistically significant, for example, a p value reaches the preset significance level for each acquisition item, sets the value to a load critical point of the corresponding acquisition item (step S2305), and terminates the processing for generating the load critical point database 110.

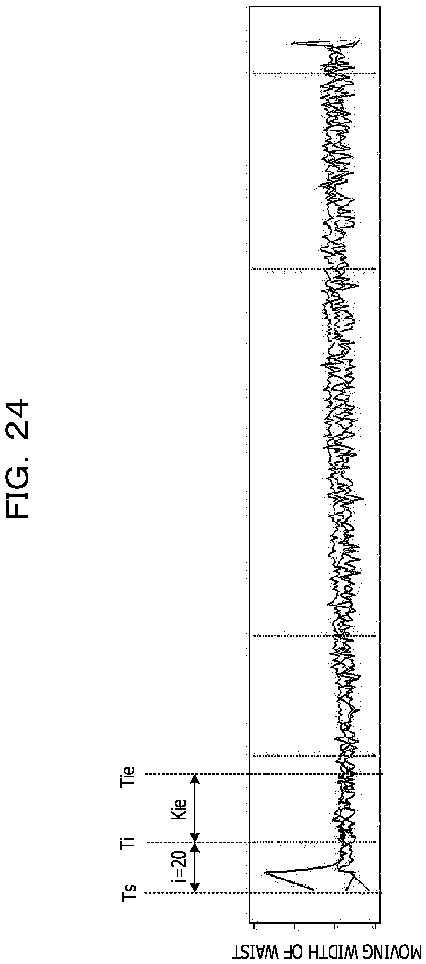

Hereinafter, the creation of the load critical point database 110 will be further described with reference to FIGs. 24 to 26.

FIG. 24 is a first diagram for explaining the creation of the load critical point database according to the first embodiment.

The graph illustrated in FIG. 24 indicates moving widths of a waist a subject in the three axis directions for one step calculated by the feature amount statistics unit 161. The moving width of the waist is one of the values indicating the motion of the upper body. In FIG. 24, it is found that the walking section starts from a time Ts.

The feature amount statistics unit 161 according to the present embodiment calculates the upper body feature amount and the leg feature amount for each section corresponding to the predetermined change width for each acquisition item.

Here, the calculation of the upper body feature amount by the feature amount statistics unit 161 in a case where the acquisition item is the "elapsed time" will be described.

In this case, since the unit of the elapsed time is [sec], the horizontal axis of the graph in FIG. 24 indicates time.

Here, the feature amount statistics unit 161 takes statistics of the moving width of the waist in a section Kie that is a predetermined section started from a time Ti that is i seconds after the start time Ts of the walking section and uses the statistics as the upper body feature amount. At this time, when it is assumed that a predetermined change width of the acquisition item "elapsed time" be one second, i = 1, 2, 3,..., 60.

For example, in a case of i = 20, the feature amount statistics unit 161 calculates an upper body feature amount in the section Kie from the time Ti that is 20 seconds after the time Ts to a time Tie. When the calculation is completed, next, the feature amount statistics unit 161 similarly calculates the upper body feature amount in a case where i = 21.

In the example in FIG. 24, since i = 60 is the maximum value, the feature amount statistics unit 161 executes similar processing until i = 60 is satisfied. Note that in the present embodiment, the length of the section Kie may be arbitrarily set. For example, the length of the section Kie is about 30 seconds.

Furthermore, for example, the calculation of the upper body feature amount by the feature amount statistics unit 161 in a case where the acquisition item is the "increase in the pulse rate" will be described.

In this case, when it is assumed that a predetermined change width of the acquisition item "increase in the pulse rate" be one [bpm] and i = 1, 2, 3,..., 40 be satisfied, the feature amount statistics unit 161 takes statistics of the moving width of the waist in the section Kie that is a predetermined section starting from a time Ti when the pulse rate is increased by one bpm from the start time Ts of the walking section and assumes the statistics as the upper body feature amount.

As described above, the critical point database creation unit 137 according to the present embodiment calculates the upper body feature amount and the leg feature amount in the section corresponding to the change width of the acquisition item for each subject.

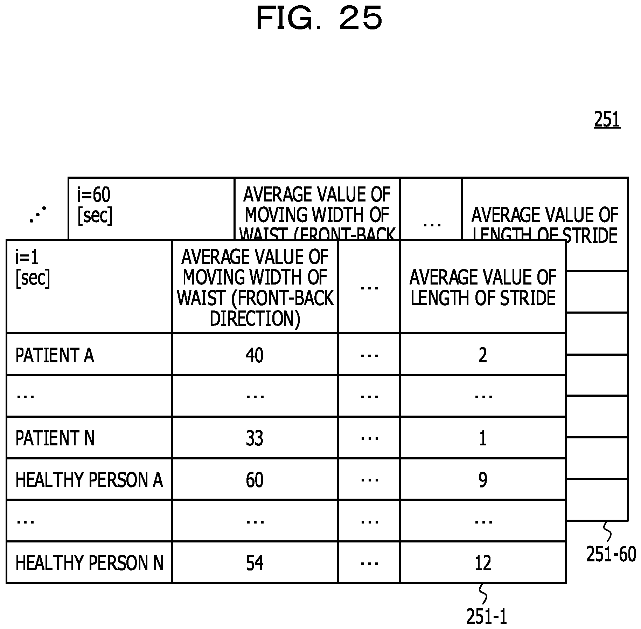

FIG. 25 is a second diagram for explaining the creation of the load critical point database according to the first embodiment.

FIG. 25 illustrates intermediate data 251 as a result of calculating the upper body feature amount and the leg feature amount in the section corresponding to the change width regarding the acquisition item "elapsed time" for each subject.

In intermediate data 251-1 in a case of i = 1, an upper body feature amount and a leg feature amount of each of patients A to N included in the concussion patient group and an upper body feature amount and a leg feature amount of each of healthy people A to N included in the healthy person group in the section Kie are calculated.

In the intermediate data 251 according to the present embodiment, up to intermediate data 251-60 in a case of i = 60, similarly, the upper body feature amount and the leg feature amount of each of the patients A to N included in the concussion patient group and the upper body feature amount and the leg feature amount of each of the healthy people A to N included in the healthy person group are calculated.

Furthermore, in the present embodiment, the intermediate data is generated for each acquisition item in the load critical point database 110.

For example, regarding the acquisition item "increase in the pulse rate", similarly, an upper body feature amount and a leg feature amount of each of the patients A to N and an upper body feature amount and a leg feature amount of each of the healthy people A to N in the section Kie in a case of i = 1 to 40 (a case where pulse rate is increased by one) are calculated.

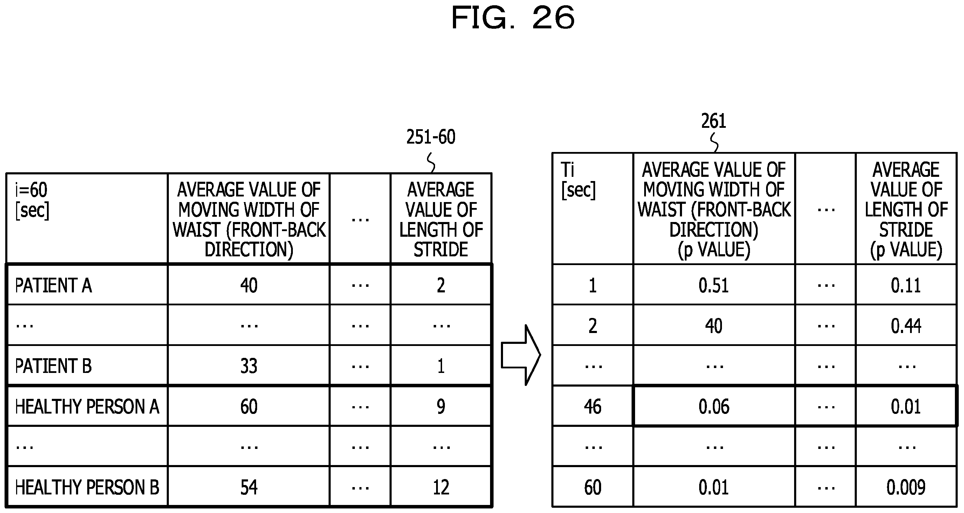

FIG. 26 is a third diagram for explaining the creation of the load critical point database according to the first embodiment.

In FIG. 26, intermediate data 261 is illustrated that indicates a result in a case where the t-test is conducted relative to the intermediate data 251 as assuming that a significance level be 0.01.

In this case, each of a p value of one leg feature amount (average value of length of stride) when the elapsed time is 46 seconds and a p value of one upper body feature amount (average value of moving width of waist in front-back direction) when the elapsed time is 60 seconds is 0.01.

Therefore, the load critical point setting unit 162 according to the present embodiment sets "46 [sec]" that is a first value from among values of the acquisition item "elapsed time" of which the p value reaches the significance level to a threshold corresponding to the acquisition item "elapsed time" in the load critical point database 110.

In the present embodiment, as described above, the threshold for each acquisition item is set, and the load critical point database 110 is generated.

Note that in the above description, the threshold for each acquisition item is set in the load critical point database 110. However, the present invention is not limited to this. For example, only the threshold "46 [sec]" corresponding to the acquisition item "elapsed time" may be stored in the load critical point database 110. In this case, a walking section that continues for 46 seconds or longer is a load section.

Furthermore, in the present embodiment, the threshold for each acquisition item may be set for each upper body feature amount and each leg feature amount.

For example, regarding the average value of the moving width of the waist in the front-back direction that is one of the upper body feature amounts, the p value reaches the significance level in a case where the elapsed time is 60 [sec]. Therefore, the threshold of the acquisition item "elapsed time" with respect to the average value of the moving width of the waist in the front-back direction is 60 [sec]. Furthermore, for example, the threshold of the acquisition item "elapsed time" with respect to the average value of the length of stride that is one of the leg feature amounts is 46 [sec].

Furthermore, in the present embodiment, the test for recognizing a significant difference between the healthy person group and the concussion patient group is conducted. However, it is possible to calculate the upper body feature amount and the leg feature amount for each change width of each acquisition item by using the sensor information 210 and 310 of the subject P and conduct the test for recognizing the significant difference with the healthy person group.

Furthermore, in the present embodiment, the load critical point of the acquisition item may be set for each individual. For example, in the present embodiment, the walking section in the past of the subject P is acquired in advance a plurality of times, and a frequency at which walking ends within a predetermined time after the "elapsed time" is calculated for each "elapsed time". For example, in a case where the "elapsed time" is one [sec] (i = 1), a frequency at which walking ends within five seconds is calculated for each acquired walking section. Furthermore, until the "elapsed time" reaches the maximum value (i = 60), the similar processing is executed.

In the present embodiment, from i = 2 to i = 60, the test is conducted to statistically check whether or not there is a significant difference between a frequency at which walking in the current "elapsed time" (i) ends and a frequency at which walking in the immediately preceding "elapsed time" (i - 1) ends. Among the values of the acquisition item "elapsed time", the "elapsed time" that is the first value in which the p value reaches the significance level is set as the threshold corresponding to the acquisition item "elapsed time". Note that probability and entropy may be used instead of the frequency.

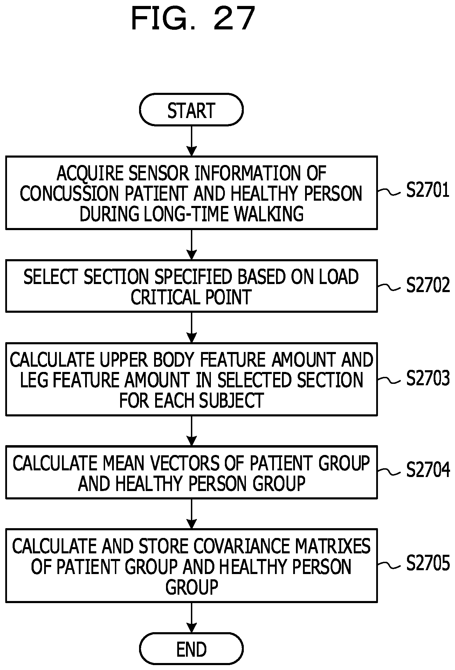

Next, the creation of the model database 120 will be described. FIG. 27 is a flowchart for explaining the processing of the model database creation unit according to the first embodiment.

The model database creation unit 138 according to the present embodiment acquires the sensor information of the concussion patient group and the sensor information of the healthy person group by the sensor information acquisition unit 131 (step S2701). The sensor information acquired here may be the same as the sensor information acquired by the critical point database creation unit 137.

Subsequently, the model database creation unit 138 selects the section specified by the section specification unit 134 in the sensor information (step S2702). Subsequently, the model database creation unit 138 calculates an upper body feature amount and a leg feature amount in the selected section by the feature amount calculation unit 135 for each subject (step S2703).

Subsequently, the model database creation unit 138 calculates a mean vector of the concussion patient group and a mean vector of the healthy person group by the mean vector calculation unit 171 (step S2704). Subsequently, the model database creation unit 138 calculates a covariance matrix of the concussion patient group and a covariance matrix of the healthy person group by the covariance matrix calculation unit 172 and stores the calculated covariance matrix in the model database 120 in association with the mean vector (step S2705), and the processing is terminated.

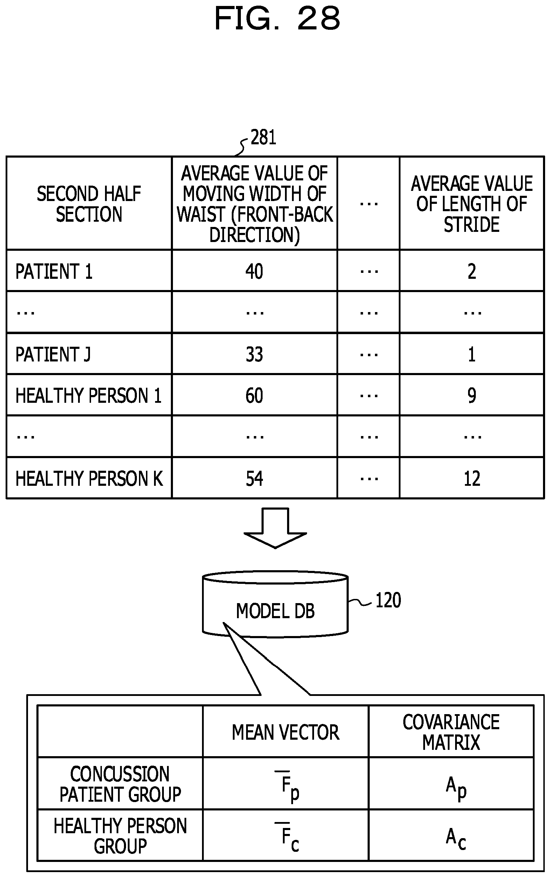

Hereinafter, the generation of the model database 120 will be further described with reference to FIG. 28. FIG. 28 is a diagram for explaining the creation of the model database according to the first embodiment.

Intermediate data 281 illustrated in FIG. 28 is generated in a case where the section half section Ka from the critical point time Tl in the load section to the end time Te of the load section is specified as the section, in which the upper body feature amount and the leg feature amount are calculated, by the section specification unit 134.

In the intermediate data 281, the model database creation unit 138 calculates the upper body feature amount and the leg feature amount of each of patients 1 to J included in the concussion patient group in the second half section in the load section acquired from the sensor information. Furthermore, similarly, in the intermediate data 281, the upper body feature amount and the leg feature amount of each of healthy people 1 to K included in the healthy person group in the second half section are calculated.

Here, the mean vector calculation unit 171 of the model database creation unit 138 according to the present embodiment calculates a mean vector for each of the concussion patient group and the healthy person group.

When it is assumed that a matrix indicating the upper body feature amount and the leg feature amount of the concussion patient group be $F_p$, $F_p$ is indicated by the following formula (3).

[Expression 3]

$$F_p = \begin{pmatrix} f_1(1) & \cdots & f_1(J) \\ f_2(1) & \cdots & f_2(J) \end{pmatrix}^T \quad , J = \text{number of patients}$$

... formula (3)

Note that at this time, $f_1(1)$ indicates one of the upper body feature amount or the leg feature amount of the subject 1. Furthermore, in the formula (3), for convenience of explanation, the upper body feature amount or the leg feature amount includes two kinds of values $f_1(J)$ and $f_2(J)$. However, in a case where the total number of the values included in the upper body feature amount and the values included in the leg feature amount is N, values up to $f_N(J)$ exist.

Furthermore, an average value of $f_1$ and $f_2$ that are one of the upper body feature amount and the leg feature amount is indicated by the following formula (4).

[Expression 4]

$$\overline{f_{p\_1}} := \frac{1}{J} \sum_{k=1}^{J} f_1(k), \quad \overline{f_{p\_2}} := \frac{1}{J} \sum_{k=1}^{J} f_2(k)$$

... formula (4)

From the formulas (3) and (4), the mean vector of the upper body feature amount and the leg feature amount of the concussion patient group is indicated by the formula (5).

[Expression 5]

$$\text{mean vector of patient group } \overline{F_p} := (\overline{f_{p\_1}}, \overline{f_{p\_2}})^T$$

... formula (5)

The mean vector calculation unit 171 according to the present embodiment similarly calculates the mean vector of the healthy person group.

Furthermore, the covariance matrix calculation unit 172 according to the present embodiment calculates the covariance matrix of the concussion patient group according to the following formulas (6) and (7).

[Expression 6]

$$B_p := F_p - \overline{F_p}$$

... formula (6)

[Expression 7]

$$\text{covariance matrix of patient group } A_p := B_p B_p{}^T$$

... formula (7)

Furthermore, the covariance matrix calculation unit 172 calculates the covariance matrix of the healthy person group by the same method.

As described above, the model database creation unit 138 according to the present embodiment calculates the mean vector and the covariance matrix of the concussion patient group and the mean vector and the covariance matrix of the healthy person group and stores the calculated values in a storage region so as to create the model database 120.

As described above, in the present embodiment, the load section in which the tendency such that the abnormality occurs in the walking state becomes strong is extracted from the walking section of the subject P, and whether or not the abnormality occurs in the walking state is estimated based on the upper body feature amount and the leg feature amount of the subject P in the load section. Therefore, according to the present embodiment, the estimation regarding whether or not the abnormality occurs based on the motion of the subject P in the walking section in which an abnormality is unlikely to occur is avoided. Therefore, according to the present embodiment, the accuracy of the estimation of the walking state can be improved.

(Second Embodiment)

Hereinafter, a second embodiment will be described with reference to the drawings. The second embodiment is different from the first embodiment in that estimation result information in consideration of the number of extracted load sections and the length of a walking time is output and a value regarding wobble is included in the upper body feature amount and the leg feature amount. Therefore, in the description of the second embodiment below, differences from the first embodiment will be described, and components having functional configurations similar to those in the first embodiment will be denoted with reference signs similar to reference sign used in the description of the first embodiment, and the description thereof will be omitted.

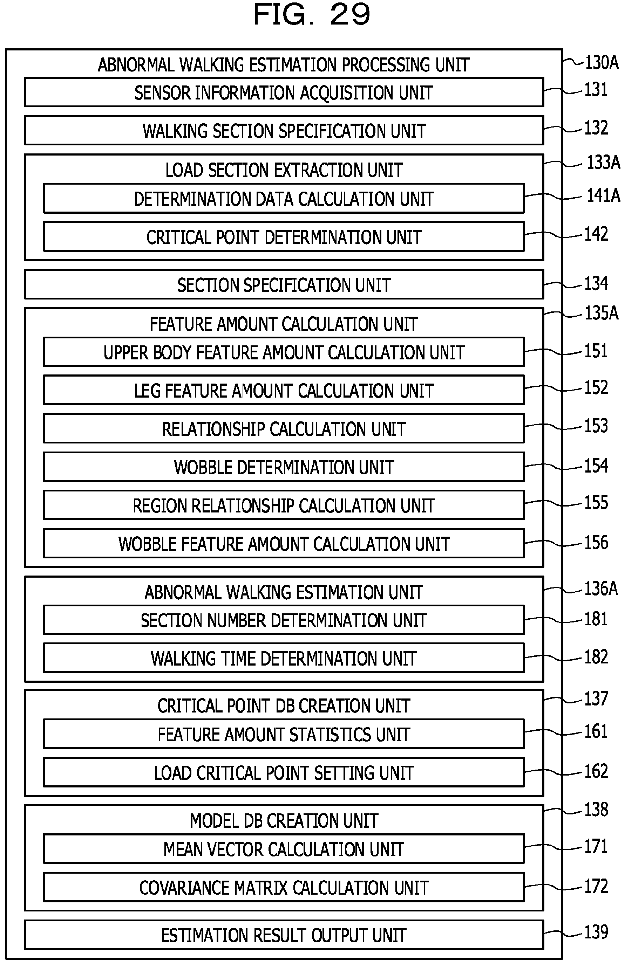

FIG. 29 is a diagram for explaining a function of an abnormal walking estimation processing unit according to the second embodiment. An abnormal walking estimation processing unit 130A according to the present embodiment includes a sensor information acquisition unit 131, a walking section specification unit 132, a load section extraction unit 133A, a section specification unit 134, a feature amount calculation unit 135A, an abnormal walking estimation unit 136A, a critical point database creation unit 137, a model database creation unit 138, and an estimation result output unit 139.

The load section extraction unit 133A according to the present embodiment includes a determination data calculation unit 141A and a critical point determination unit 142. The determination data calculation unit 141A according to the present embodiment includes the number of times of wobbles of a subject P and a walking distance in items of a determination vector of the subject P.

More specifically, when the subject P repeatedly wobbles, the determination data calculation unit 141A acquires a value indicating a relation between normal walking and the repetition of the wobbles and may include the value as elements of the determination vector.

In that case, it is preferable that an item corresponding to the value indicating the relation between the normal walking and the repetition of the wobbles and a threshold thereof be stored in the acquisition items in the load critical point database 110. The relation between the normal walking and the repetition of the wobbles will be described later in detail.

The feature amount calculation unit 135A according to the present embodiment includes an upper body feature amount calculation unit 151, a leg feature amount calculation unit 152, a relationship calculation unit 153, a wobble determination unit 154, a region relationship calculation unit 155, and a wobble feature amount calculation unit 156.

In a case where the wobble determination unit 154 determines that the subject P wobbles, the wobble feature amount calculation unit 156 according to the present embodiment may calculate generation ratios of an instantaneous feature amount and a continuous feature amount in the specified section (refer to FIG. 18) and include the result as the upper body feature amount.

The generation ratio of the instantaneous feature amount is calculated by "the number of instantaneous feature amounts in the section specified by the section specification unit 134/the number of values of the moving width of the waist in the vertical direction in the section specified by the section specification unit 134". Furthermore, the generation ratio of the continuous feature amount is calculated by "the number of continuous feature amounts in the section specified by the section specification unit 134/the number of values of the moving width of the waist in the vertical direction in the section specified by the section specification unit 134".

The abnormal walking estimation unit 136A according to the present embodiment includes a section number determination unit 181 and a walking time determination unit 182.

The section number determination unit 181 according to the present embodiment determines whether or not the number of sections extracted as the load section is equal to or more than a predetermined number. Note that the predetermined number is a value set in advance, and for example, may be set by a medical worker or the like who uses an information processing apparatus 100.

The walking time determination unit 182 according to the present embodiment determines whether or not a walking time acquired from sensor information 210 and 310 is equal to or longer than a predetermined time. Note that the predetermined time is a value set in advance, and for example, may be set by a medical worker or the like who uses the information processing apparatus 100.

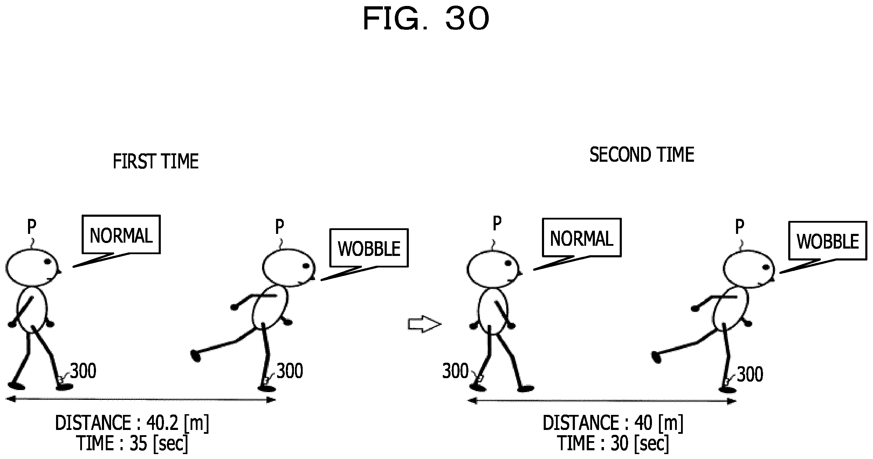

Hereinafter, the relation between the normal walking and the repetition of the wobbles will be described with reference to FIG. 30. FIG. 30 is a diagram for explaining the relation between the normal walking and the repetition of wobbles according to the second embodiment.

The determination data calculation unit 141A according to the present embodiment acquires a total number of times when the normal walking is switched to the wobble state and average values of the walking distances and the elapsed times until the wobble occurs as values indicating the relation between the normal walking and the repetition of the wobbles. In FIG. 30, a case where the wobble occurs twice is illustrated.

In FIG. 30, a first wobble occurs at the time when an elapsed time from the start of walking is 35 [sec] and a walking distance from the start of walking is 40.2 [m]. Then, a second wobble occurs at the time when an elapsed time from the start of walking is 30 [sec] and a walking distance from the start of walking is 40 [m].

In this case, the determination data calculation unit 141A calculates "twice" as the total number of times, "40.1 [m]" as the average value of the walking distances, and "32.5 [sec]" as the average value of the elapsed times. Then, the determination data calculation unit 141A includes these values as the elements of the determination vector.

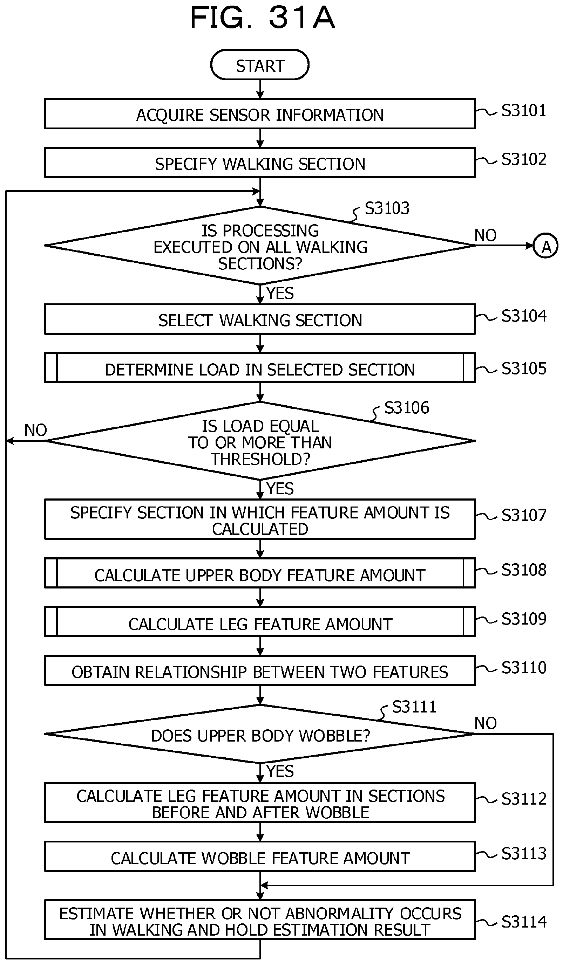

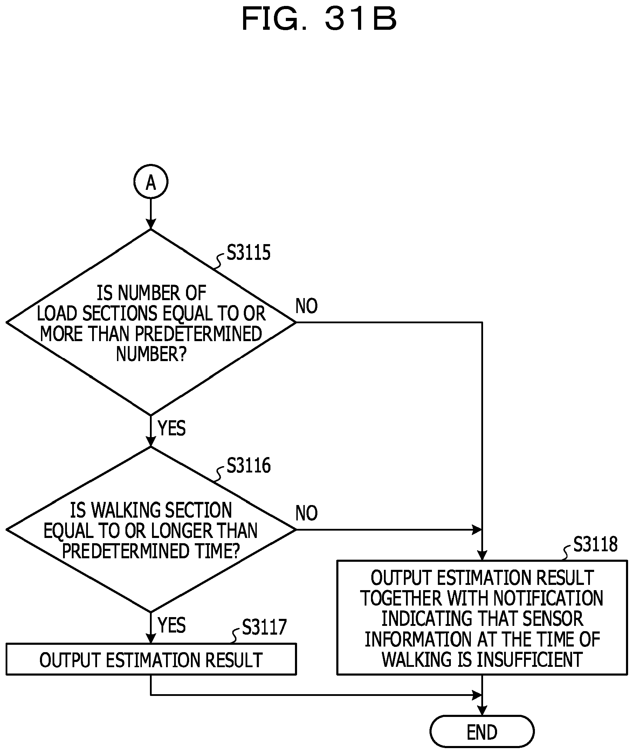

Next, an operation of the abnormal walking estimation processing unit 130A according to the present embodiment will be described with reference to FIG. 31. FIG. 31 is a flowchart for explaining processing of the abnormal walking estimation processing unit according to the second embodiment.

Since processing from step S3101 to step S3112 in FIG. 31 is similar to the processing from step S701 to step S712 in FIG. 7, the description thereof will be omitted.

Subsequent to step S3112, the abnormal walking estimation processing unit 130A calculates a value to be included in the upper body feature amount based on the wobble by the wobble feature amount calculation unit 156 (step S3113).

Specifically, the wobble feature amount calculation unit 156 calculates a generation ratio of an instantaneous feature value and a generation ratio of a continuous feature amount and includes these values as the upper body feature amount.

Subsequently, the abnormal walking estimation processing unit 130A estimate whether or not an abnormality occurs in the walking state of the subject P by using the upper body feature amount and the leg feature amount by the abnormal walking estimation unit 136A and holds the results (step S3114), and the procedure returns to step S3103.

In a case where the processing is executed on all the walking sections in step S3103, the abnormal walking estimation processing unit 130A determines whether or not the number of load sections extracted by the load section extraction unit 133 is equal to or more than a predetermined number by the section number determination unit 181 of the abnormal walking estimation unit 136A (step S3115).

In a case where the number of load sections is not equal to or more than the predetermined number in step S3115, that is, in a case where the number of load sections extracted by the load section extraction unit 133 is less than the predetermined number, the abnormal walking estimation processing unit 130A proceeds the procedure to step S3118 described later.

In a case where the number of load sections is equal to or more than the predetermined number in step S3115, the abnormal walking estimation processing unit 130A determines whether or not a walking time that is the sum of walking times in all the walking sections extracted by the walking section specification unit 132 is equal to or longer than a predetermined time by the walking time determination unit 182 (step S3116).

In a case where the walking time is equal to or longer than the predetermined time in step S3116, the abnormal walking estimation unit 136A outputs the estimation result (step S3117), and the processing is terminated.

When the walking time is not equal to or longer than the predetermined time in step S3116, that is, when the walking time is shorter than the predetermined time, the abnormal walking estimation unit 136A notifies that the sensor information at the time of walking is insufficient and outputs estimation result information (step S3118), and the processing is terminated.

In this way, in the present embodiment, in a case where it is determined that the wobble occurs, the values related to the wobble can be included in the upper body feature amount.

Furthermore, for example, in a case where the number of load sections used for estimation of the walking state is small and in a case where the walking time is short, there is a possibility that the sufficient estimation result cannot be obtained.

Therefore, in the present embodiment, the number of load sections required for estimation of the walking state and the walking time are respectively set as the predetermined number and the predetermined time. Then, in a case where the number of load sections is less than the predetermined number and in a case where the walking time is shorter than the predetermined time, the notification indicating that the sensor information during walking is insufficient is output together with the estimation result information.

Moreover, in a case where the subject P is a severe concussion patient, there is a possibility that walking is interrupted before the item of the determination vector reaches the threshold. Therefore, in the present embodiment, for example, the notification such that "A ratio such that the walking time is less than the predetermined time is 500. There is high possibility that long-time (long-distance) walking is difficult for the subject P" may be output.

Furthermore, in the present embodiment, in a case where the number of load sections is less than the predetermined number, it is possible to calculate a feature amount in a load section extracted by using a threshold set for each individual and estimate whether or not an abnormality occurs by the using the model database.

Note that for example, the medical worker and the like who use the information processing apparatus 100 may arbitrarily set the predetermined number and the predetermined time.

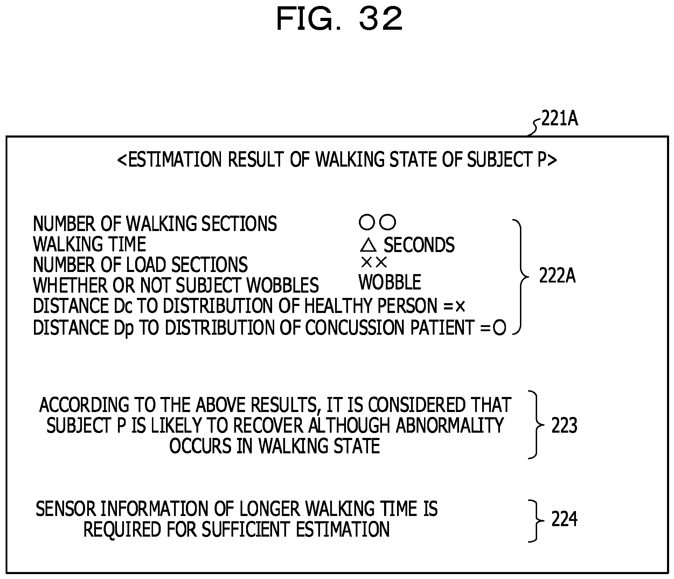

FIG. 32 is a diagram illustrating an exemplary output of the estimation result information according to the second embodiment. In a screen 221A illustrated in FIG. 32, information 222A indicating the result of the processing that is the basis of the estimation by the abnormal walking estimation processing unit 130 and information 223 indicating the estimation result are displayed as the estimation result information.

Furthermore, a notification 224 indicating that the sensor information during walking is insufficient is displayed on the screen 221A.

The information 222A according to the present embodiment includes information 225 indicating the walking time in addition to the number of extracted walking sections, the number of load sections, whether or not the subject P wobbles, the Mahalanobis Distance ($D_p$, $D_c$) as the estimation result of the walking state of the subject P.

As described above, in the present embodiment, by displaying the notification 224 indicating that the sensor information during walking is insufficient together with the estimation result information, for example, it is possible to prompt the medical worker or the like and the subject P to acquire further sensor information during walking. Furthermore, according to the present embodiment, by displaying the notification 224 together with the estimation result information, the medical worker and the like can grasp reliability of the estimation result information.

(Third Embodiment)

Hereinafter, a third embodiment will be described with reference to the drawings. The third embodiment is different from the first embodiment in that a terminal device acquires sensor information 210 and 310 and transmits the sensor information 210 and 310 to an information processing apparatus. Therefore, in the invention of the third embodiment below, only differences from the first embodiment will be described, and components having functional configurations similar to those in the first embodiment will be denoted with reference signs similar to reference sign used in the description of the first embodiment, and the description thereof will be omitted.

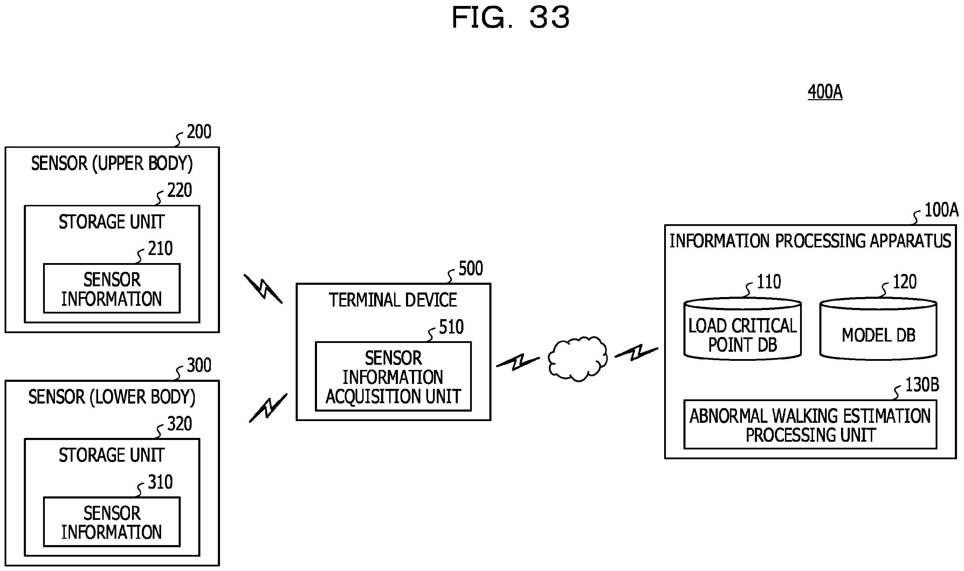

FIG. 33 is a diagram illustrating an exemplary system configuration of an information processing system according to the third embodiment. An information processing system 400A according to the present embodiment includes an information processing apparatus 100A, sensors 200 and 300, and a terminal device 500.

In the information processing system 400A according to the present embodiment, the information processing apparatus 100A is connected to the terminal device 500 via a network. Additionally, in the information processing system 400A according to the present embodiment, the terminal device 500 and the sensors 200 and 300 are connected by wireless communication or the like. Note that the terminal device 500 and the sensors 200 and 300 may also be connected by wire.

The information processing apparatus 100A according to the present embodiment includes a load critical point database 110, a model database 120, and an abnormal walking estimation processing unit 130B.

The abnormal walking estimation processing unit 130B according to the present embodiment is different from the abnormal walking estimation processing unit 130 according to the first embodiment only in a point that the sensor information acquisition unit 131 acquires the sensor information 210 and 310 from the terminal device 500.

The terminal device 500 according to the present embodiment may also be, for example, a smartphone, a tablet terminal, or the like, or may be a communication device including a memory device and a communication function.

The terminal device 500 according to the present embodiment includes a sensor information acquisition unit 510, and acquires the pieces of sensor information 210 and 310 stored respectively in a storage unit 220 of the sensor 200 and a storage unit 320 of the sensor 300, and the sensors 200 and 300 are connected to the terminal device 500.

In the present embodiment, when the terminal device 500 acquires the pieces of sensor information 210 and 310, the terminal device 500 transmits the pieces of sensor information 210 and 310 to the information processing apparatus 100A. The information processing apparatus 100A receives the sensor information 210 and 310 and estimates the walking state of the subject P by the abnormal walking estimation processing unit 130B.

Furthermore, the abnormal walking estimation processing unit 130B according to the present embodiment transmits the estimation result information to the terminal device 500 by the estimation result output unit 139.

When receiving the estimation result information, the terminal device 500 displays the estimation result information on a display and the like.

In this way, in the present embodiment, since the sensor information 210 and 310 is transmitted from the terminal device 500 to the information processing apparatus 100A, for example, the embodiment can be applied to a case where the information processing apparatus 100A is a server and the like provided on a web.

Note that in the present embodiment, the abnormal walking estimation processing unit 130B specifies the walking section from the sensor information. However, the present invention is not limited to this. For example, in the present embodiment, the terminal device 500 may specify the walking section of the subject, and the abnormal walking estimation processing unit 130B may receive the sensor information in a state where the walking section is specified. In that case, it is sufficient that the abnormal walking estimation processing unit 130B start the processing from the processing of the load section extraction unit 133.

Moreover, in the present embodiment, if the walking section was specified, when receiving only the sensor information 210, the abnormal walking estimation unit 136 can estimate the walking state by only the upper body feature amount calculated by the upper body feature amount calculation unit 151 and the relationship calculation unit 153. Furthermore, in a case where the walking section is not specified, the walking section can be specified by using the sensor attached to the waist.

The present invention is not limited to the embodiments specifically disclosed above, and various modifications and changes can be made without departing from the scope of the claims.

REFERENCE SIGNS LIST

| | |
|---|---|
| 100, 100A | information processing apparatus |
| 110 | load critical point database |
| 120 | model database |
| 130, 130A, 130B | abnormal walking estimation processing unit |
| 131 | sensor information acquisition unit |
| 132 | walking section specification unit |
| 133 | load section extraction unit |
| 134 | section specification unit |
| 135, 135A | feature amount calculation unit |
| 136, 136A | abnormal walking estimation unit |
| 137 | critical point database creation unit |
| 138 | model database creation unit |
| 141 | determination data calculation unit |
| 142 | critical point determination unit |
| 151 | upper body feature amount calculation unit |
| 152 | leg feature amount calculation unit |
| 153 | relationship calculation unit |
| 154 | wobble determination unit |
| 155 | region relationship calculation unit |
| 156 | wobble feature amount calculation unit |
| 200, 300 | sensor |
| 210, 310 | sensor information |
| 400, 400A | information processing system |
| 500 | terminal device |

**Claims**

1. An information processing apparatus (100) comprising:

a walking section specification unit (132) configured to specify (S702) a walking section in which a subject walks from sensor information (210, 310) indicating a motion of the subject;
a load section extraction unit (133) configured to:

calculate (S705), from the sensor information (210, 310), an elapsed time from the start of the walking of the subject, an increase in the pulse rate of the subject, an accumulation of a number of times of wobble of the subject, and a walking distance of the subject as values of items;
compare (S706) each of the values of items with respective thresholds; and
extract (S707) a time point when at least one of the values of items is equal to or greater than the respective threshold as a start time of a load section in which a physical load is applied to the subject;

a feature amount calculation unit (135) configured to calculate (S708, S709) a feature amount indicating the motion of the subject in the load section; and
an estimation unit (136) configured to estimate (S713) a physical state of the subject based on the feature amount.

2. The information processing apparatus (100) according to claim 1, wherein the physical state includes an abnormal state different from a normal state and a recovery state in which the state approaches the normal state with time.

3. The information processing apparatus (100) according to claim 1, wherein the load section extraction unit (133) includes:

a determination data calculation unit (141) that calculates the values of items using the sensor information (210, 310) as elements in time series in the walking section; and

a determination unit (142) that refers to a storage unit (100) that stores the threshold for each item and determines whether the values of items are equal to or greater than the respective thresholds.

4. The information processing apparatus (100) according to claim 3, further comprising a database creation unit (137) configured to acquire sensor information indicating a motion of each subject from a first subject group and a second subject group and calculate the threshold for each item, wherein the database creation unit (137) calculates

a feature amount indicating a motion in a predetermined section after the value of the item changes for each subject included in the first subject group, and

a feature amount indicating a motion in a predetermined section after the value of the item changes for each subject included in the second subject group, and

sets the value of the item when a significant difference is recognized between the first subject group and the second subject group as a threshold corresponding to the item.

5. The information processing apparatus (100) according to claim 4, wherein in a case where a frequency at which the walking section ends is high in the predetermined section after the value of the item changes, the value of the item is set as the threshold corresponding to the item.

6. The information processing apparatus (100) according to claim 5, wherein

the sensor information (210, 310) includes first sensor information (210) acquired from a first sensor (200) attached to an upper body of the subject, and

the feature amount calculation unit (135) calculates an upper body feature amount indicating a feature of a motion of the upper body of the subject by using the first sensor information (210).

7. The information processing apparatus (100) according to claim 6, wherein

the sensor information includes second sensor information (310) acquired from a second sensor (300) attached to both legs of the subject, and

the feature amount calculation unit (135) calculates a leg feature amount indicating motions of the both legs of the subject by using the second sensor information (310).

8. The information processing apparatus (100) according to claim 6 or 7, further comprising a section specification unit (134) configured to specify a section in which the upper body and/or leg feature amounts of the subject is calculated by the feature amount calculation unit (135) in the load section, wherein

in the load section, a section from a time when any one of elements included in the values of items reaches the threshold of the corresponding item to an end time of the load section is specified as a section in which the upper body feature amount and the leg feature amount are calculated.

9. The information processing apparatus (100) according to claim 1, further comprising:

an action time determination unit (182) configured to determine whether or not a walking time of all the walking sections specified by the walking section specification unit (132) is less than a predetermined time;

a section number determination unit (181) configured to determine whether or not the number of load sections is less than a predetermined number; and

an estimation result output unit (139) configured to output a notification indicating that the sensor information (210, 310) is insufficient together with an estimation result by the estimation unit in a case where the walking time is shorter than a predetermined time or in a case where the number of load sections is less than a predetermined number.

10. The information processing apparatus (100) according to claim 3, wherein the load section extraction unit (133) multiplies the threshold for each item by a predetermined value by using a state before the walking section of the subject specified from the sensor information (210, 310) and compares the obtained value with the values of items.

11. An information processing system (400) including a sensor (200, 300) that detects a motion of a subject whose physical state is estimated and an information processing apparatus (100) according to any one of claims 1 to 10.

12. A computer-implemented information processing method, wherein the computer:

specifies (S702) a walking section in which a subject walks from sensor information (210, 310) indicating a motion of the subject;
calculates (S705), from the sensor information (210, 310), an elapsed time from the start of the walking of the subject, an increase in the pulse rate of the subject, an accumulation of a number of times of wobble of the subject, and a walking distance of the subject as values of items;
compares (S706) each of the values of items with respective thresholds;
extracts (S707) a time point when at least one of the values of items is equal to or greater than the respective threshold as a start time of a load section in which a physical load is applied to the subject;
calculates (S708, S709) a feature amount indicating the motion of the subject in the load section; and
estimates (S713) a physical state of the subject based on the feature amount.

**Patentansprüche**

1. Informationsverarbeitungsvorrichtung (100), umfassend:

eine Gehabschnitt-Spezifikationseinheit (132), die dazu ausgelegt ist, einen Gehabschnitt, in dem eine Person geht, aus Sensorinformationen (210, 310), die eine Bewegung der Person angeben, zu spezifizieren (S702);
eine Lastabschnitt-Extraktionseinheit (133), die ausgelegt ist zum:

Berechnen (S705), aus den Sensorinformationen (210, 310), einer verstrichenen Zeit seit Beginn des Gehens der Person, einer Erhöhung der Pulsfrequenz der Person,
einer Häufung einer Anzahl von Wacklern der Person, und einer Gehentfernung der Person als Werte von Elementen;
Vergleichen (S706) jedes der Werte von Elementen mit entsprechenden Schwellenwerten; und
Extrahieren (S707) eines Zeitpunkts, wenn mindestens einer der Werte von Elementen gleich oder größer als der jeweilige Schwellenwert ist, als eine Startzeit eines Lastabschnitts, in dem eine physische Belastung auf das Person ausgeübt wird;

eine Merkmalsbetrag-Berechnungseinheit (135), die dazu ausgelegt ist, einen Merkmalsbetrag, der die Bewegung der Person in dem Lastabschnitt angibt, zu berechnen (S708, S709); und
eine Schätzeinheit (136), die dazu ausgelegt ist, einen körperlichen Zustands der Person basierend auf dem Merkmalsbetrag zu schätzen (S713).

2. Informationsverarbeitungsvorrichtung (100) nach Anspruch 1, wobei der physikalische Zustand einen anormalen Zustand, der sich von einem normalen Zustand unterscheidet, und einen Wiederherstellungszustand, in dem sich der Zustand mit der Zeit dem normalen Zustand annähert, aufweist.

3. Informationsverarbeitungsvorrichtung (100) nach Anspruch 1, wobei die Lastabschnitt-Extraktionseinheit (133) aufweist:

eine Bestimmungsdaten-Berechnungseinheit (141), die die Werte von Elementen unter Verwendung der Sensorinformationen (210, 310) als Elemente in Zeitreihen in dem Gehabschnitt berechnet; und
eine Bestimmungseinheit (142), die sich auf eine Speichereinheit (100) bezieht, die den Schwellenwert für jedes Element speichert, und bestimmt, ob die Werte der Elemente gleich oder größer als die jeweiligen Schwellenwerte sind.

4. Informationsverarbeitungsvorrichtung (100) nach Anspruch 3, ferner umfassend eine Datenbankerzeugungseinheit (137), die dazu ausgelegt ist, Sensorinformationen zu erfassen, die eine Bewegung jeder Person einer ersten Personengruppe und einer zweiten Personengruppe angeben, und den Schwellenwert für jedes Element berechnen, wobei die Datenbank-Erzeugungseinheit (137) ist, zum Berechnen,

eines Merkmalsbetrags, der eine Bewegung in einem vorbestimmten Abschnitt angibt, nachdem sich der Wert des Elements geändert hat, für jede Person, die in der ersten Personengruppe enthalten ist, und eines Merkmalsbetrags, der eine Bewegung in einem vorbestimmten Abschnitt angibt, nachdem sich der Wert des Elements geändert hat, für jede Person, die in der zweiten Personengruppe enthalten ist, und Einstellen des Wert des Elements, wenn ein signifikanter Unterschied zwischen der ersten Personengruppe und der zweiten Personengruppe erkannt wird, als ein dem Element entsprechender Schwellenwert.

5. Informationsverarbeitungsvorrichtung (100) nach Anspruch 4, wobei in einem Fall, in dem eine Häufigkeit, mit der der Gehabschnitt endet, in dem vorbestimmten Abschnitt hoch ist, nachdem sich der Wert des Elements geändert hat, der Wert des Elements als der dem Element entsprechende Schwellenwert eingestellt wird.

6. Informationsverarbeitungsvorrichtung (100) nach Anspruch 5, wobei die Sensorinformationen (210, 310) erste Sensorinformationen (210) aufweisen, die von einem ersten Sensor (200) erfasst werden, der an einem Oberkörper der Person angebracht ist, und die Merkmalsbetrag-Berechnungseinheit (135) einen Oberkörper-Merkmalsbetrag, der ein Merkmal einer Bewegung des Oberkörpers der Person angibt, unter Verwendung der ersten Sensorinformationen (210) berechnet.

7. Informationsverarbeitungsvorrichtung (100) nach Anspruch 6, wobei die Sensorinformationen zweite Sensorinformationen (310) aufweisen, die von einem zweiten Sensor (300) erfasst werden, der an beiden Beinen der Person angebracht ist, und die Merkmalsbetrags-Berechnungseinheit (135) einen Bein-Merkmalsbetrag, der Bewegungen der beiden Beine der Person angibt, unter Verwendung der zweiten Sensorinformationen (310) berechnet.

8. Informationsverarbeitungsvorrichtung (100) nach Anspruch 6 oder 7, ferner umfassend eine Abschnittspezifizierungseinheit (134), die dazu ausgelegt ist, einen Abschnitt zu spezifizieren, in dem die Oberkörper- und/oder Bein-Merkmalsbeträge der Person durch die Merkmalbetrags-Berechnungseinheit (135) in dem Lastabschnitt berechnet werden, wobei in dem Lastabschnitt ein Abschnitt von einem Zeitpunkt, wenn eines der Elemente, das in den Werten von Elementen enthalten ist, den Schwellenwert des entsprechenden Elements erreicht, bis zu einem Endzeitpunkt des Lastabschnitts als ein Abschnitt spezifiziert wird, in dem der Oberkörper-Merkmalsbetrag und der Bein-Merkmalsbetrag berechnet werden.

9. Informationsverarbeitungsvorrichtung (100) nach Anspruch 1, ferner umfassend:

eine Aktionszeit-Bestimmungseinheit (182), die dazu ausgelegt ist, zu bestimmen, ob eine Gehzeit aller Gehabschnitte, die durch die Gehabschnitt-Spezifikationseinheit (132) spezifiziert sind, kleiner als eine vorbestimmte Zeit ist oder nicht;
eine Abschnittsanzahl-Bestimmungseinheit (181), die dazu ausgelegt ist, zu bestimmen, ob die Anzahl von Lastabschnitten kleiner als eine vorbestimmte Anzahl ist oder nicht; und
eine Schätzergebnis-Ausgabeeinheit (139), die dazu ausgelegt ist, eine Benachrichtigung auszugeben, die anzeigt, dass die Sensorinformationen (210, 310) unzureichend sind,
zusammen mit einem Schätzergebnis durch die Schätzeinheit, in einem Fall, in dem die Gehzeit kürzer als eine vorbestimmte Zeit ist, oder in einem Fall, in dem die Anzahl von Lastabschnitten kleiner als eine vorbestimmte Anzahl ist.

10. Informationsverarbeitungsvorrichtung (100) nach Anspruch 3, wobei die Lastabschnitt-Extraktionseinheit (133) den Schwellenwert für jedes Element mit einem vorbestimmten Wert multipliziert, indem sie einen Zustand vor dem Gehabschnitt der Person verwendet, der aus den Sensorinformationen (210, 310) spezifiziert wird, und den erhaltenen Wert mit den Werten der Elemente vergleicht.

11. Informationsverarbeitungssystem (400), aufweisend einen Sensor (200, 300), der eine Bewegung einer Person erkennt, deren physikalischer Zustand geschätzt wird, und eine Informationsverarbeitungsvorrichtung (100) nach einem der Ansprüche 1 bis 10.

12. Computerimplementiertes Informationsverarbeitungsverfahren, bei dem der Computer ist zum:

Spezifizieren (S702) eines Gehabschnitt, in dem eine Person geht, aus Sensorinformationen (210, 310), die eine Bewegung der Person angeben;
Berechnen (S705), aus den Sensorinformationen (210, 310), einer verstrichenen Zeit seit Beginn des Gehens der Person, einer Erhöhung der Pulsfrequenz der Person, einer Häufung einer Anzahl von Wacklern der Person , und einer Gehentfernung der Person als Werte von Elementen;

Vergleichen (S706) jedes der Werte von Elementen mit entsprechenden Schwellenwerten;
Extrahieren (S707) eines Zeitpunkts, wenn mindestens einer der Werte von Elementen gleich oder größer als der jeweilige Schwellenwert ist, als eine Startzeit eines Lastabschnitts, in dem eine physische Belastung auf das Person ausgeübt wird;
Berechnen (S708, S709) eines Merkmalsbetrags, der die Bewegung der Person in dem Lastabschnitt angibt; und
Schätzen (S713) eines körperlichen Zustands der Person basierend auf dem Merkmalsbetrag.

**Revendications**

1. Appareil de traitement d'informations (100) comprenant :

   une unité de spécification de section de marche (132) configurée pour spécifier (S702) une section de marche dans laquelle un sujet marche à partir d'informations de capteur (210, 310) indiquant un mouvement du sujet ;
   une unité d'extraction de section de charge (133) configurée pour :

   calculer (S705), à partir des informations de capteur (210, 310), un temps écoulé depuis le début de la marche du sujet, une augmentation du pouls du sujet, une accumulation d'un certain nombre d'oscillations du sujet, et une distance de marche du sujet en tant que valeurs d'éléments ;
   comparer (S706) chacune des valeurs d'éléments à des seuils respectifs ; et
   extraire (S707) un instant où au moins l'une des valeurs des éléments est supérieure ou égale au seuil respectif en tant qu'instant de départ d'une section de charge dans laquelle une charge physique est appliquée au sujet ;
   une unité de calcul de quantité de caractéristique (135) configurée pour calculer (S708, S709) une quantité de caractéristique indiquant le mouvement du sujet dans la section de charge ; et
   une unité d'estimation (136) configurée pour estimer (S713) un état physique du sujet sur la base de la quantité de caractéristique.

2. Appareil de traitement d'informations (100) selon la revendication 1, dans lequel l'état physique comporte un état anormal différent d'un état normal et un état de récupération dans lequel l'état se rapproche de l'état normal avec le temps.

3. Appareil de traitement d'informations (100) selon la revendication 1, dans lequel l'unité d'extraction de section de charge (133) comporte :

   une unité de calcul de données de détermination (141) qui calcule les valeurs d'éléments à l'aide des informations de capteur (210, 310) en tant qu'éléments en séries temporelles dans la section de marche ; et
   une unité de détermination (142) qui fait référence à une unité de stockage (100) qui stocke le seuil pour chaque élément et
   détermine si les valeurs des éléments sont supérieures ou égales aux seuils respectifs.

4. Appareil de traitement d'informations (100) selon la revendication 3, comprenant en outre une unité de création de base de données (137) configurée pour acquérir des informations de capteur indiquant un mouvement de chaque sujet à partir d'un premier groupe de sujets et d'un second groupe de sujets et calculer le seuil pour chaque élément, dans lequel l'unité de création de base de données (137) calcule
   une quantité de caractéristique indiquant un mouvement dans une section prédéterminée après que la valeur de l'élément change pour chaque sujet inclus dans le premier groupe de sujets, et une quantité de caractéristique indiquant un mouvement dans une section prédéterminée après que la valeur de l'élément change pour chaque sujet inclus dans le second groupe de sujets, et définit la valeur de l'élément lorsqu'une différence significative est reconnue entre le premier groupe de sujets et le second groupe de sujets comme seuil correspondant à l'élément.

5. Appareil de traitement d'informations (100) selon la revendication 4, dans lequel dans un cas où une fréquence à laquelle la section de marche se termine est élevée dans la section prédéterminée après que la valeur de l'élément change, la valeur de l'élément est définie comme le seuil correspondant à l'élément.

6. Appareil de traitement d'informations (100) selon la revendication 5, dans lequel
   les informations de capteur (210, 310) comportent des premières informations de capteur (210) acquises à partir d'un premier capteur (200) attaché sur le haut du corps du sujet, et l'unité de calcul de quantité de caractéristique (135) calcule une quantité de caractéristique du haut du corps indiquant une caractéristique d'un mouvement du haut du corps du sujet en utilisant les premières informations de capteur (210).

7. Appareil de traitement d'informations (100) selon la revendication 6, dans lequel

les informations de capteur comportent des secondes informations de capteur (310) acquises à partir d'un second capteur (300) attaché aux deux jambes du sujet, et
l'unité de calcul de quantité de caractéristique (135) calcule une quantité de caractéristique de jambe indiquant les mouvements des deux jambes du sujet en utilisant les secondes informations de capteur (310).

8. Appareil de traitement d'informations (100) selon la revendication 6 ou 7, comprenant en outre une unité de spécification de section (134) configurée pour spécifier une section dans laquelle le haut du corps et/ou les quantités de caractéristique de jambe du sujet sont calculées par l'unité de calcul de quantité de caractéristique (135) dans la section de charge, dans lequel

dans la section de charge, une section à partir d'un instant où l'un quelconque des éléments inclus dans les valeurs des éléments atteint le seuil de l'élément correspondant jusqu'à un instant de fin de la section de charge est spécifiée comme une section dans laquelle la quantité de caractéristique du haut du corps et la quantité de caractéristique de jambe sont calculées.

9. Appareil de traitement d'informations (100) selon la revendication 1, comprenant en outre :

une unité de détermination de temps d'action (182) configurée pour déterminer si oui ou non un temps de marche de toutes les sections de marche spécifiées par l'unité de spécification de section de marche (132) est inférieur à un temps prédéterminé ;
une unité de détermination de numéro de section (181) configurée pour déterminer si oui ou non le nombre de sections de charge est inférieur à un nombre prédéterminé ; et
une unité de sortie de résultat d'estimation (139) configurée pour émettre une notification indiquant que les informations de capteur (210, 310) sont insuffisantes conjointement avec un résultat d'estimation par l'unité d'estimation dans un cas où le temps de marche est plus court qu'un temps prédéterminé ou
dans un cas où le nombre de sections de charge est inférieur à un nombre prédéterminé.

10. Appareil de traitement d'informations (100) selon la revendication 3, dans lequel l'unité d'extraction de section de charge (133) multiplie le seuil pour chaque élément par une valeur prédéterminée en utilisant un état avant la section de marche du sujet spécifié à partir des informations de capteur (210, 310) et compare la valeur obtenue aux valeurs des éléments.

11. Système de traitement d'informations (400) comportant un capteur (200, 300) qui détecte un mouvement d'un sujet dont l'état physique est estimé et un appareil de traitement d'informations (100) selon l'une quelconque des revendications 1 à 10.

12. Procédé de traitement d'informations mis en œuvre par ordinateur, dans lequel l'ordinateur :

spécifie (S702) une section de marche dans laquelle un sujet marche à partir d'informations de capteur (210, 310) indiquant un mouvement du sujet ;
calcule (S705), à partir des informations de capteur (210, 310), un temps écoulé depuis le début de la marche du sujet, une augmentation du pouls du sujet, une accumulation d'un certain nombre d'oscillations du sujet, et une distance de marche du sujet en tant que valeurs d'éléments ;
compare (S706) chacune des valeurs d'éléments à des seuils respectifs ;
extrait (S707) un instant où au moins l'une des valeurs des éléments est supérieure ou égale au seuil respectif en tant qu'instant de départ d'une section de charge dans laquelle une charge physique est appliquée au sujet ;
calcule (S708, S709) une quantité de caractéristique indiquant le mouvement du sujet dans la section de charge ; et
estime (S713) un état physique du sujet sur la base de la quantité de caractéristique.

# FIG. 1

INFORMATION PROCESSING APPARATUS ~100

SENSOR INFORMATION (UPPER BODY) ~210

P

200

SENSOR INFORMATION (LOWER BODY) ~310

300

VERTICAL DIRECTION
(Z-AXIS DIRECTION)

FRONT-BACK DIRECTION
(Y-AXIS DIRECTION)

LATERAL DIRECTION
(X-AXIS DIRECTION)

EP 3 626 169 B1

# FIG. 2

SENSOR (UPPER BODY) 200
STORAGE UNIT 220
SENSOR INFORMATION 210

SENSOR (LOWER BODY) 300
STORAGE UNIT 320
SENSOR INFORMATION 310

400

INFORMATION PROCESSING APPARATUS 100
LOAD CRITICAL POINT DB 110
MODEL DB 120
ABNORMAL WALKING ESTIMATION PROCESSING UNIT 130

# FIG. 3

<u>200</u>

```
┌──────────┐   ┌──────────────┐   ┌──────────┐
│  MEMORY  │15 │  ARITHMETIC  │16 │INTERFACE │17
│  DEVICE  │   │  PROCESSING  │   │  DEVICE  │
│          │   │   DEVICE     │   │          │
└────┬─────┘   └──────┬───────┘   └────┬─────┘
     ↕                ↕                 ↕
B ───┼────────────────┼─────────────────┼────────
     ↕          ↕          ↕          ↕
┌─────────┐11 ┌────────┐12 ┌──────────┐13 ┌──────────┐14
│  INPUT  │   │ OUTPUT │   │  DRIVE   │   │ AUXILIARY│
│ DEVICE  │   │ DEVICE │   │ DEVICE   │   │ STORAGE  │
│         │   │        │   │          │   │ DEVICE   │
└─────────┘   └────────┘   └────┬─────┘   └──────────┘
                                ↕
                           ┌──────────┐18
                           │RECORDING │
                           │ MEDIUM   │
                           └──────────┘
```

# FIG. 4

110

| ACQUISITION ITEM | THRESHOLD |
|---|---|
| ELAPSED TIME [sec] | 46 |
| INCREASE IN PULSE RATE [bpm] | 30 |
| ... | ... |
| MOVING DISTANCE [m] | 10 |

# FIG. 5

120

| | MEAN VECTOR | COVARIANCE MATRIX |
|---|---|---|
| CONCUSSION PATIENT GROUP | $\overline{F}_p$ | $A_p$ |
| HEALTHY PERSON GROUP | $\overline{F}_c$ | $A_c$ |

# FIG. 6

| ABNORMAL WALKING ESTIMATION PROCESSING UNIT | ~130 |
|---|---|

| SENSOR INFORMATION ACQUISITION UNIT | ~131 |
|---|---|

| WALKING SECTION SPECIFICATION UNIT | ~132 |
|---|---|

| LOAD SECTION EXTRACTION UNIT | ~133 |
|---|---|

| DETERMINATION DATA CALCULATION UNIT | ~141 |
|---|---|

| CRITICAL POINT DETERMINATION UNIT | ~142 |
|---|---|

| SECTION SPECIFICATION UNIT | ~134 |
|---|---|

| FEATURE AMOUNT CALCULATION UNIT | ~135 |
|---|---|

| UPPER BODY FEATURE AMOUNT CALCULATION UNIT | ~151 |
|---|---|

| LEG FEATURE AMOUNT CALCULATION UNIT | ~152 |
|---|---|

| RELATIONSHIP CALCULATION UNIT | ~153 |
|---|---|

| WOBBLE DETERMINATION UNIT | ~154 |
|---|---|

| REGION RELATIONSHIP CALCULATION UNIT | ~155 |
|---|---|

| ABNORMAL WALKING ESTIMATION UNIT | ~136 |
|---|---|

| CRITICAL POINT DB CREATION UNIT | ~137 |
|---|---|

| FEATURE AMOUNT STATISTICS UNIT | ~161 |
|---|---|

| LOAD CRITICAL POINT SETTING UNIT | ~162 |
|---|---|

| MODEL DB CREATION UNIT | ~138 |
|---|---|

| MEAN VECTOR CALCULATION UNIT | ~171 |
|---|---|

| COVARIANCE MATRIX CALCULATION UNIT | ~172 |
|---|---|

| ESTIMATION RESULT OUTPUT UNIT | ~139 |
|---|---|

FIG. 7

```
                    ┌─────────┐
                    │  START  │
                    └────┬────┘
                         │
        ┌────────────────────────────────┐
        │   ACQUIRE SENSOR INFORMATION    │──S701
        └────────────────┬───────────────┘
                         │
        ┌────────────────────────────────┐
        │    SPECIFY WALKING SECTION      │──S702
        └────────────────┬───────────────┘
                         │
                       ◇ S703
          IS PROCESSING EXECUTED ON ALL    YES
             WALKING SECTIONS?        ────────────┐
                         │ NO                      │
                       S704                     S714
        ┌────────────────────────────────┐   ┌──────────────────────────────┐
        │    SELECT WALKING SECTION       │   │   OUTPUT ESTIMATION RESULT    │
        └────────────────┬───────────────┘   └──────────────┬───────────────┘
                         │                                   │
        ┌────────────────────────────────┐              ┌────────┐
        │ DETERMINE LOAD IN SELECTED       │──S705        │  END   │
        │         SECTION                 │              └────────┘
        └────────────────┬───────────────┘
                         │
                       ◇ S706
     NO      IS LOAD EQUAL TO OR MORE THAN
    ◄────          THRESHOLD?
                         │ YES
        ┌────────────────────────────────┐
        │  SPECIFY SECTION IN WHICH        │──S707
        │  FEATURE AMOUNT IS CALCULATED    │
        └────────────────┬───────────────┘
                         │
        ┌────────────────────────────────┐
        │  CALCULATE UPPER BODY            │──S708
        │      FEATURE AMOUNT             │
        └────────────────┬───────────────┘
                         │
        ┌────────────────────────────────┐
        │ CALCULATE LEG FEATURE AMOUNT     │──S709
        └────────────────┬───────────────┘
                         │
        ┌────────────────────────────────┐
        │ OBTAIN RELATIONSHIP BETWEEN      │──S710
        │      TWO FEATURES               │
        └────────────────┬───────────────┘
                         │
                       ◇ S711
            DOES UPPER BODY WOBBLE?     NO
                         │ YES   ──────────┐
        ┌────────────────────────────────┐ │
        │ CALCULATE LEG FEATURE AMOUNT IN  │──S712
        │ SECTIONS BEFORE AND AFTER WOBBLE │ │
        └────────────────┬───────────────┘ │
                         ◄──────────────────┘
        ┌────────────────────────────────┐
        │ ESTIMATE WHETHER OR NOT          │──S713
        │ ABNORMALITY OCCURS IN WALKING    │
        │ AND HOLD ESTIMATION RESULT       │
        └────────────────┬───────────────┘
```

# FIG. 8A

# FIG. 8B

# FIG. 8C

EP 3 626 169 B1

# FIG. 9

```
        ┌──────────────────────────┐
        │   FROM STEP S704         │
        │   IN FIG. 7              │
        └──────────────────────────┘
                    │
                    ▼
    ┌──────────────────────────────────────┐
    │ ACQUIRE DETERMINATION DATA REGARDING  │
    │ LOAD DURING WALKING (SIGNAL REGARDING │ ～ S901
    │ LOAD ELAPSED TIME, PULSE RATE,...)    │
    └──────────────────────────────────────┘
                    │
                    ▼
    ┌──────────────────────────────────────┐
    │ COMPARE WITH THRESHOLD STORED IN LOAD │ ～ S902
    │ CRITICAL POINT DB                     │
    └──────────────────────────────────────┘
                    │
                    ▼
        ┌──────────────────────────┐
        │   PROCEED TO STEP S706   │
        │   IN FIG. 7              │
        └──────────────────────────┘
```

# FIG. 10

| ACQUISITION ITEM | t1 | t2 | ⋯ | t(n-1) | tn |
|---|---|---|---|---|---|
| ELAPSED TIME [sec] | 1 | 2 | ⋯ | 46 | 47 |
| INCREASE IN PULSE RATE [bpm] | 1 | 2 | ⋯ | 10 | 12 |
| ⋯ | ⋯ | ⋯ | ⋯ | ⋯ | ⋯ |
| MOVING DISTANCE [m] | 5 | 10 | ⋯ | 50 | ⋯ |

| ACQUISITION ITEM | THRESHOLD |
|---|---|
| ELAPSED TIME [sec] | 46 |
| INCREASE IN PULSE RATE [bpm] | 30 |
| ⋯ | ⋯ |
| MOVING DISTANCE [m] | 10 |

COMPARE AT EACH TIME

EP 3 626 169 B1

# FIG. 11

# FIG. 12

# FIG. 13

FROM STEP S707
IN FIG. 7

CALCULATE LOCUS OF WAIST FOR EACH TWO STEPS — S1301

CALCULATE MOVING WIDTHS OF WAIST IN
THREE AXIS DIRECTIONS — S1302

S1303

IS PROCESSING
EXECUTED ON ALL STEPS IN SPECIFIED
SECTION?

NO

YES

TAKE STATISTICS OF MOVING WIDTH IN SPECIFIED
SECTION AND HOLD RESULT — S1304

PROCEED TO STEP S1401
IN FIG. 14

# FIG. 14

FROM STEP S1304
IN FIG. 13

CALCULATE ANGLES OF WAIST IN FRONT-BACK
DIRECTION AND LATERAL DIRECTION — S1401

TAKE STATISTICS OF ACQUIRED ANGLES OF WAIST — S1402

PROCEED TO STEP S709
IN FIG. 7

# FIG. 15

# FIG. 16

```
┌─────────────────────┐
│  FROM STEP S708     │
│  IN FIG. 7          │
└─────────────────────┘
          │
          ▼
┌─────────────────────────────────┐
│ CALCULATE FEATURE AMOUNT        │── S1601
│ INDICATING MOTION OF LEG        │
└─────────────────────────────────┘
          │
          ▼
       S1602
   ◇ IS PROCESSING EXECUTED
     ON ALL STEPS IN SECTION? ◇── NO
          │ YES
          ▼
┌─────────────────────────────────┐
│ TAKE STATISTICS OF FEATURE      │
│ AMOUNT INDICATING MOTION OF LEG │── S1603
│ AND HOLD RESULT AS LEG FEATURE  │
│ AMOUNT                          │
└─────────────────────────────────┘
          │
          ▼
┌─────────────────────┐
│ PROCEED TO STEP S710│
│ IN FIG.7            │
└─────────────────────┘
```

# FIG. 17

# FIG. 18

# FIG. 19

FIG. 20A          FIG. 20B          FIG. 20C

EP 3 626 169 B1

# FIG. 21

DISTRIBUTION OF
CONCUSSION PATIENT
B1

B2
DISTRIBUTION OF
HEALTHY PERSON

f

Dc

Dp

AVERAGE VALUE OF
MOVING WIDTH OF WAIST

AVERAGE VALUE OF ANGLE OF WAIST

# FIG. 22

221

<ESTIMATION RESULT OF WALKING STATE OF SUBJECT P>

NUMBER OF WALKING SECTIONS     ○○
NUMBER OF LOAD SECTIONS     ××
WHETHER OR NOT SUBJECT WOBBLES    WOBBLE
DISTANCE Dc TO DISTRIBUTION OF HEALTHY PERSON = ×
DISTANCE Dp TO DISTRIBUTION OF CONCUSSION PATIENT = ○

222

ACCORDING TO THE ABOVE RESULTS, IT IS CONSIDERED THAT
SUBJECT P IS LIKELY TO RECOVER ALTHOUGH ABNORMALITY
OCCURS IN WALKING STATE

223

# FIG. 23

START

ACQUIRE SENSOR INFORMATION OF CONCUSSION PATIENT GROUP AND SENSOR INFORMATION OF HEALTHY PERSON GROUP — S2301

CALCULATE VALUE INDICATING MOTION FOR EACH STEP OF EACH SUBJECT BY USING SENSOR INFORMATION — S2302

TAKE STATISTICS OF VALUE INDICATING MOTION IN SECTION CORRESPONDING TO PREDETERMINED CHANGE WIDTH FOR EACH ACQUISITION ITEM AND HOLD RESULTS AS UPPER BODY FEATURE AMOUNT AND LEG FEATURE AMOUNT — S2303

CONDUCT TEST OF SIGNIFICANT DIFFERENCE BETWEEN GROUPS REGARDING UPPER BODY FEATURE AMOUNT AND LEG FEATURE AMOUNT IN EACH SECTION FOR EACH ACQUISITION ITEM — S2304

SET FIRST VALUE WHEN p VALUE REACHES PRESET SIGNIFICANCE LEVEL TO LOAD CRITICAL POINT OF EACH ACQUISITION ITEM — S2305

END

# FIG. 24

MOVING WIDTH OF WAIST

# FIG. 25

251

| ∴ | i=60<br>[sec] | AVERAGE VALUE OF<br>MOVING WIDTH OF<br>WAIST (FRONT-BACK | ... | AVERAGE VALUE OF<br>LENGTH OF STRIDE | |
|---|---|---|---|---|---|
| i=1<br>[sec] | | AVERAGE VALUE OF<br>MOVING WIDTH OF<br>WAIST (FRONT-BACK<br>DIRECTION) | ... | AVERAGE VALUE OF<br>LENGTH OF STRIDE | |
| PATIENT A | | 40 | ... | 2 | |
| ... | | ... | ... | ... | |
| PATIENT N | | 33 | ... | 1 | |
| HEALTHY PERSON A | | 60 | ... | 9 | |
| ... | | ... | ... | ... | |
| HEALTHY PERSON N | | 54 | ... | 12 | |

251-60

251-1

# FIG. 26

251-60

| i=60 [sec] | AVERAGE VALUE OF MOVING WIDTH OF WAIST (FRONT-BACK DIRECTION) | ... | AVERAGE VALUE OF LENGTH OF STRIDE |
|---|---|---|---|
| PATIENT A | 40 | ... | 2 |
| ... | ... | ... | ... |
| PATIENT B | 33 | ... | 1 |
| HEALTHY PERSON A | 60 | ... | 9 |
| ... | ... | ... | ... |
| HEALTHY PERSON B | 54 | ... | 12 |

261

| Ti [sec] | AVERAGE VALUE OF MOVING WIDTH OF WAIST (FRONT-BACK DIRECTION) (p VALUE) | ... | AVERAGE VALUE OF LENGTH OF STRIDE (p VALUE) |
|---|---|---|---|
| 1 | 0.51 | ... | 0.11 |
| 2 | 40 | ... | 0.44 |
| ... | ... | ... | ... |
| 46 | 0.06 | ... | 0.01 |
| ... | ... | ... | ... |
| 60 | 0.01 | ... | 0.009 |

EP 3 626 169 B1

# FIG. 27

```
START
   │
   ▼
```

ACQUIRE SENSOR INFORMATION OF
CONCUSSION PATIENT AND HEALTHY PERSON
DURING LONG-TIME WALKING   —— S2701

```
   │
   ▼
```

SELECT SECTION SPECIFIED BASED ON LOAD
CRITICAL POINT   —— S2702

```
   │
   ▼
```

CALCULATE UPPER BODY FEATURE AMOUNT AND
LEG FEATURE AMOUNT IN SELECTED SECTION
FOR EACH SUBJECT   —— S2703

```
   │
   ▼
```

CALCULATE MEAN VECTORS OF PATIENT GROUP
AND HEALTHY PERSON GROUP   —— S2704

```
   │
   ▼
```

CALCULATE AND STORE COVARIANCE MATRIXES
OF PATIENT GROUP AND HEALTHY PERSON
GROUP   —— S2705

```
   │
   ▼
  END
```

# FIG. 28

281

| SECOND HALF SECTION | AVERAGE VALUE OF MOVING WIDTH OF WAIST (FRONT-BACK DIRECTION) | ... | AVERAGE VALUE OF LENGTH OF STRIDE |
|---|---|---|---|
| PATIENT 1 | 40 | ... | 2 |
| ... | ... | ... | ... |
| PATIENT J | 33 | ... | 1 |
| HEALTHY PERSON 1 | 60 | ... | 9 |
| ... | ... | ... | ... |
| HEALTHY PERSON K | 54 | ... | 12 |

MODEL DB ～120

| | MEAN VECTOR | COVARIANCE MATRIX |
|---|---|---|
| CONCUSSION PATIENT GROUP | $\overline{F}_p$ | $A_p$ |
| HEALTHY PERSON GROUP | $\overline{F}_c$ | $A_c$ |

# FIG. 29

| ABNORMAL WALKING ESTIMATION PROCESSING UNIT | ~130A |
|---|---|

| SENSOR INFORMATION ACQUISITION UNIT | ~131 |
|---|---|

| WALKING SECTION SPECIFICATION UNIT | ~132 |
|---|---|

**LOAD SECTION EXTRACTION UNIT** — 133A

| DETERMINATION DATA CALCULATION UNIT | ~141A |
|---|---|
| CRITICAL POINT DETERMINATION UNIT | ~142 |

| SECTION SPECIFICATION UNIT | ~134 |
|---|---|

**FEATURE AMOUNT CALCULATION UNIT** — 135A

| UPPER BODY FEATURE AMOUNT CALCULATION UNIT | ~151 |
|---|---|
| LEG FEATURE AMOUNT CALCULATION UNIT | ~152 |
| RELATIONSHIP CALCULATION UNIT | ~153 |
| WOBBLE DETERMINATION UNIT | ~154 |
| REGION RELATIONSHIP CALCULATION UNIT | ~155 |
| WOBBLE FEATURE AMOUNT CALCULATION UNIT | ~156 |

**ABNORMAL WALKING ESTIMATION UNIT** — 136A

| SECTION NUMBER DETERMINATION UNIT | ~181 |
|---|---|
| WALKING TIME DETERMINATION UNIT | ~182 |

**CRITICAL POINT DB CREATION UNIT** — 137

| FEATURE AMOUNT STATISTICS UNIT | ~161 |
|---|---|
| LOAD CRITICAL POINT SETTING UNIT | ~162 |

**MODEL DB CREATION UNIT** — 138

| MEAN VECTOR CALCULATION UNIT | ~171 |
|---|---|
| COVARIANCE MATRIX CALCULATION UNIT | ~172 |

| ESTIMATION RESULT OUTPUT UNIT | ~139 |
|---|---|

# FIG. 30

# FIG. 31A

START

ACQUIRE SENSOR INFORMATION ~ S3101

SPECIFY WALKING SECTION ~ S3102

S3103
IS PROCESSING EXECUTED ON ALL WALKING SECTIONS? — NO → Ⓐ

YES

SELECT WALKING SECTION ~ S3104

DETERMINE LOAD IN SELECTED SECTION ~ S3105

S3106
IS LOAD EQUAL TO OR MORE THAN THRESHOLD? — NO →

YES

SPECIFY SECTION IN WHICH FEATURE AMOUNT IS CALCULATED ~ S3107

CALCULATE UPPER BODY FEATURE AMOUNT ~ S3108

CALCULATE LEG FEATURE AMOUNT ~ S3109

OBTAIN RELATIONSHIP BETWEEN TWO FEATURES ~ S3110

S3111
DOES UPPER BODY WOBBLE? — NO

YES

CALCULATE LEG FEATURE AMOUNT IN SECTIONS BEFORE AND AFTER WOBBLE ~ S3112

CALCULATE WOBBLE FEATURE AMOUNT ~ S3113

ESTIMATE WHETHER OR NOT ABNORMALITY OCCURS IN WALKING AND HOLD ESTIMATION RESULT ~ S3114

# FIG. 31B

A

S3115
IS NUMBER OF
LOAD SECTIONS EQUAL TO OR
MORE THAN PREDETERMINED
NUMBER?
NO

YES

S3116
IS WALKING SECTION
EQUAL TO OR LONGER THAN
PREDETERMINED TIME?
NO

YES S3117
OUTPUT ESTIMATION RESULT

S3118
OUTPUT ESTIMATION RESULT
TOGETHER WITH NOTIFICATION
INDICATING THAT SENSOR
INFORMATION AT THE TIME OF
WALKING IS INSUFFICIENT

END

# FIG. 32

221A

<ESTIMATION RESULT OF WALKING STATE OF SUBJECT P>

NUMBER OF WALKING SECTIONS　　　　○○
WALKING TIME　　　　　　　　　　　△ SECONDS
NUMBER OF LOAD SECTIONS　　　　　××
WHETHER OR NOT SUBJECT WOBBLES　　WOBBLE
DISTANCE Dc TO DISTRIBUTION OF HEALTHY PERSON =×
DISTANCE Dp TO DISTRIBUTION OF CONCUSSION PATIENT =○

222A

ACCORDING TO THE ABOVE RESULTS, IT IS CONSIDERED THAT
SUBJECT P IS LIKELY TO RECOVER ALTHOUGH ABNORMALITY
OCCURS IN WALKING STATE

223

SENSOR INFORMATION OF LONGER WALKING TIME IS
REQUIRED FOR SUFFICIENT ESTIMATION

224

## FIG. 33

SENSOR (UPPER BODY) ⌐200

   STORAGE UNIT ⌐220

      SENSOR INFORMATION ⌐210

SENSOR (LOWER BODY) ⌐300

   STORAGE UNIT ⌐320

      SENSOR INFORMATION ⌐310

TERMINAL DEVICE ⌐500

   SENSOR INFORMATION ACQUISITION UNIT ⌐510

400A

INFORMATION PROCESSING APPARATUS ⌐100A

LOAD CRITICAL POINT DB ⌐110

MODEL DB ⌐120

ABNORMAL WALKING ESTIMATION PROCESSING UNIT ⌐130B

EP 3 626 169 B1

**EP 3 626 169 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013110010 A1
- EP 1466557 A2
- JP 2016106848 A
- JP 2010110399 A
- JP 2011177278 A